# EUROPEAN PATENT SPECIFICATION

(11) **EP 0 403 250 B1**
(45) Date of publication and mention of the grant of the patent: **23.11.1994**
(21) Application number: 90306439.2
(22) Date of filing: 13.06.1990
(51) Int. Cl.: C07D 303/48, C07C 323/56, C07C 319/14, A61K 31/19

(54) **Phenylglycidamides useful for preparing alpha-hydroxy-beta-sulfido-arylpropionic acid derivatives**
Phenylglycidamide, verwendbar in der Herstellung von alpha-Hydroxy-beta-sulfido-arylpropionsäure-Derivaten
Phénylglycinamides utiles dans la préparation de dérivés d'acides alpha-hydroxy-bêta-sulfido-arylpropioniques

(30) Priority: 14.06.1989 US 366078
(43) Date of publication of application: 19.12.1990
(73) Proprietor: SMITHKLINE BEECHAM CORPORATION, Philadelphia Pennsylvania 19101 (US)
(72) Inventor: Jarmas, Alvydas Alfonsas, Wynnewood, Pennsylvania 19096 (US); Novack, Vance John, Devon, Pennsylvania 19333 (US)
(74) Representative: Giddings, Peter John, Dr.

(56) References cited:
- EP-A- 0 202 759
- US-A- 4 341 718
- JOURNAL OF ORGANIC CHEMISTRY, vol. 28, no. 6, June 1963, pages 1514-1521,Washington, DE, US; C.C. TUNG et al.: "The darzens condensation. II. Reaction of chloroacetamides with aromatic aldehydes"
- JOURNAL OF ORGANIC CHEMISTRY, vol. 50, no. 9, 3rd May 1985, pages 1560-1563,Washington, DC, US; J.M. CHONG et al.: "Nucleophilic openings of 2,3-epoxyacids and amides mediated by Ti(O-i-Pr)4. Reliable C-3 selectivity"

## Description

### Field of the Invention

This invention relates to novel intermediates for the preparation of certain leukotriene antagonists.

### Background of the Invention

Leukotrienes are a group of physiologically important mediators. In particular, the peptido-leukotrienes are believed to mediate such processes as edema formation and bronchoconstriction, and are believed to play a role in allergic conditions, asthma and a number of other pathophysiological conditions in mammals. Antagonists of the peptido-leukotrienes have been developed and are useful in the treatment of conditions which are mediated by these compounds. Certain of these antagonists are characterized by being α-hydroxy, β-aryl, β-sulfido propionic acids, amides and esters, and are disclosed, for example, in EP-A-0 375 348, EP-A 0 313 697, EP-A 0 358 240, EP-A 0 365 149, and EP-A 0 296 731, all of which are incorporated herein by reference. Generally, these antagonists are represented by formula (I): 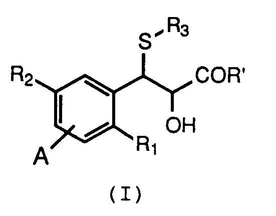
wherein:
R₁ is (L)ₐ-(CH₂)_{b}-(T)_{c}-B;
a is 0 or 1;
b is 3 to 14;
c is 0 or 1;
L and T are independently sulfur, oxygen, CH=CH, C≡C, or CH₂;
B is H, C₁₋₄alkyl, ethynyl, trifluoromethyl, isopropenyl, furanyl, thienyl, cyclohexyl or phenyl optionally monosubstituted with Br, Cl, CF₃, C₁₋₄alkoxy, C₁₋₄alkyl, methylthio or trifluoromethylthio;
R′ is OH, NH₂, aryloxy or C₁₋₆alkoxy;
R₂ and A are independently selected from H, CF₃, C₁₋₄alkyl, C₁₋₄alkoxy, F, Cl, Br, I, OH, NO₂ or NH₂; or, when R₁ and A are H, R₂ is (L)ₐ-(CH₂)_{b}-(T)_{c}-B wherein a, b, c, L, T and B are as defined above;
R³ is (CH₂)ₙCH(R₅)COR₆, CH(CO₂H)CH₂CO₂H, CH₂CH₂Z, 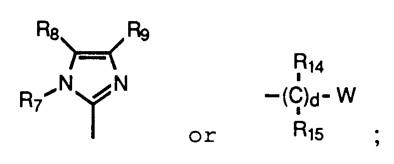
n is 0 to 6;
R₅ is hydrogen, amino, or NHCOCH₂CH₂CH(NH₂)CO₂H;
R₆ is hydroxy, amino, NHCH₂CO₂H or C₁₋₆alkoxy;
Z is SO₃H, SO₂NH₂ or CN;
R₇ is hydrogen, C₁₋₄alkyl or C₃₋₄alkenyl;
R₈ is hydrogen, C₁₋₄alkyl, carboxyl, carboxamido, or (CH₂)ₚCO₂R₁₂, wherein p is 1 or 2 and R₁₂ is C₁₋₆alkyl or hydrogen when R₇ and R₉ are hydrogen or C₁₋₄alkyl;
R₉ is hydrogen, C₁₋₄alkyl, or (CH₂)ₚCO₂R₁₃, wherein p is 1 or 2 and R₁₃ is C₁₋₆alkyl or hydrogen, with the proviso that when n is 0, R₅ is hydrogen and further that R₇, R₈ and R₉ are not all hydrogen;
R₁₄ and R₁₅ are independently hydrogen or C₁₋₄alkyl at any point when d is not 0;
d is 0 to 6;
W is a six membered aryl or heteroaryl ring selected from phenyl, pyridyl, or pyrimidyl, unsubstituted or substituted with F, E, or D; or W is one of 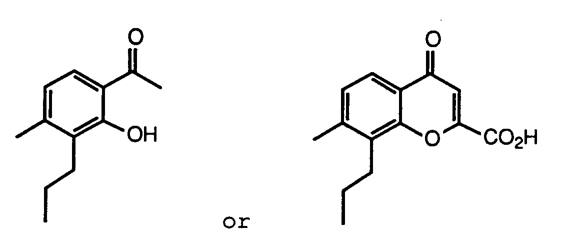
F is 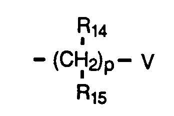
wherein R₁₄ and R₁₅ are independently hydrogen or C₁₋₄alkyl;
p is 0 to 6;
V is H, C₁₋₄alkyl, COR′, SO₃H, SO₂H, SO₂NH₂, COCH₂OH, CHOHCH₂OH, or tetrazolyl, with R′ as defined above; and
E and D are independently selected from H, OH, F, Cl, Br, CF₃, C₁₋₄alkyl, C₁₋₄alkoxy, methylthio, trifluoromethylthio, NO₂, NH₂, NHC₁₋₄alkyl, or C₁₋₄alkylCO-; or pharmaceutically acceptable salts thereof.

Processes for preparing compounds of formula (I) generally consist of the condensation of a mercaptan, such as R₃-SH, wherein R₃ is as defined above, with an epoxide of the formula (II): 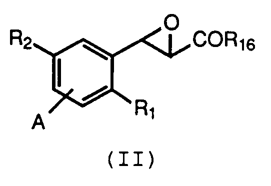
wherein R₁, R₂ and A are as defined above, and R₁₆ is C₁₋₆alkoxy.

Such reactions are commonly conducted in an alcoholic solvent in the presence of a base, such as triethylamine. However, the nucleophilic opening of the epoxide treated in this manner is generally regio random, proceeding to yield a mixture of α-hydroxy, β-sulfido and α-sulfido, β-hydroxy esters as regioisomers as depicted in Scheme A. (See Gleason et al., J.Med. Chem., 30, 959(1987).) This is in contrast to the nucleophilic opening of allylic epoxides, which occurs exclusively α to the unsaturated bond. (See Corey et al., J. Am. Chem Soc., 102(4), 1436-9 (1980).) 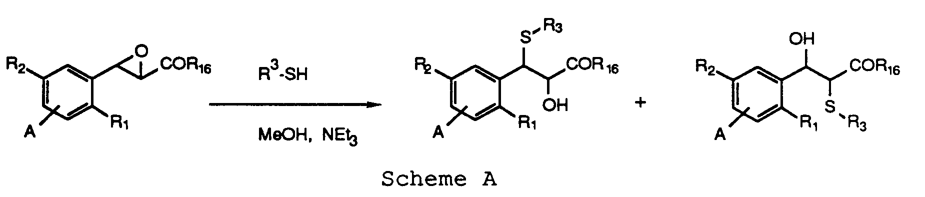
Lack of regio control results in low yields of the desired α-hydroxy, β-sulfido isomer and greatly increases manufacturing costs. No suitable methods for producing the compounds of formula (I) from the α-epoxy esters of formula (II) have been disclosed. A method for producing a regioselective opening of the epoxy intermediates given by formula (II) is therefore desirable.

Methods of controlling the regioselectivity of epoxide openings have been disclosed for certain α-epoxy acids, α-epoxy alcohols and α-epoxy amides. (See Chong, et al., J. Org. Chem., 50, 1560 (1985).) Chong et al. disclose that, in the presence of titanium isopropoxide, aliphiatic α-epoxy acids and secondary amides, when reacted with thiophenol, diethylamine, cyanide or azide, show a preference for opening the epoxide in the β-position. Such reactions have not been shown for primary amides or the aryl α-epoxy esters, amides or carboxylic acids of this invention. In additon, the combination of conversion of α-epoxy esters to α-epoxy carboxamides, followed by regioselective epoxide opening and conversion to esters or acids in order to effect β-addition of α-epoxy esters has not been reported.

β-substituted glycidamides of formula (III), 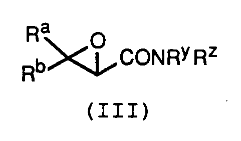
wherein R^{a} is methyl, ethyl, phenyl, phenoxymethylene or benzyl, R^{b} is H, methyl or ethyl, and R^{y} and R^{z} are H or methyl, have been reported by Fourneau et al., J. Pharm. Chim., 19, 49(1934). The compounds therein in which R^{a} is phenyl, have no ortho or meta substituents corresponding to those in the instant invention.

Fourneau et al., Bull. Soc. Chim., 7, 593(1940), also disclose β-phenyl glycidamides of formula (III), wherein R^{a} is H, R^{b} is phenyl, R^{y} is H or methyl and R^{z} is H, methyl or benzyl. None of the β-phenyl glycidamides disclosed possess substitution on the phenyl ring corresponding to the instant invention.

### Summary of the Invention

In one respect, this invention is intermediate compounds for the production of pharmaceutically important leukotriene antagonists, according to the formula (IV). 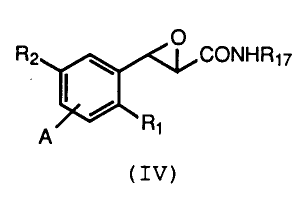
wherein:
R₁ is (L)ₐ-(CH₂)_{b}-(T)_{c}-B;
a is 0 or 1;
b is 3 to 14;
c is 0 or 1;
L and T are independently sulfur, oxygen, CH=CH, C≡C, or CH₂;
B is H, C₁₋₄alkyl, ethynyl, trifluoromethyl, isopropenyl, furanyl, thienyl, cyclohexyl or phenyl optionally monosubstituted with Br, Cl, CF₃, C₁₋₄alkoxy, C₁₋₄alkyl, methylthio or trifluoromethylthio;
R₂ and A are independently selected from H, CF₃, C₁₋₄alkyl, C₁₋₄alkoxy, F, Cl, Br, I, OH, NO₂ or NH₂; or, when R₁ and A are H, R₂ is (L)ₐ-(CH₂)_{b}-(T)_{c}-B wherein a, b, c, L, T and B are as defined above;
R₁₇ is H, phenyl, benzyl or C₁₋₄alkyl.

In another respect, this invention is a process for preparing compounds of formula (I), as hereinbefore defined, by reacting a compound of formula (IV) with a mercaptide R₃-SM, wherein R₃ is as defined for formula (I) and M is an alkali metal, in the presence of titanium isopropoxide.

### Detailed Description of the Invention

This invention relates to compounds of the formula (IV): 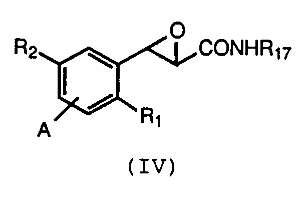
wherein:
R₁ is (L)ₐ-(CH₂)_{b}-(T)_{c}-B;
a is 0 or 1;
b is 3 to 14;
c is 0 or 1;
L and T are independently sulfur, oxygen, CH=CH, C≡C, or CH₂;
B is H, C₁₋₄alkyl, ethynyl, trifluoromethyl, isopropenyl, furanyl, thienyl, cyclohexyl or phenyl optionally monosubstituted with Br, Cl, CF₃, C₁₋₄alkoxy, C₁₋₄alkyl, methylthio or trifluoromethylthio;
R₂ and A are independently selected from H, CF₃, C₁₋₄alkyl, C₁₋₄alkoxy, F, Cl, Br, I, OH, NO₂ or NH₂; or, when R₁ and A are H, R₂ is (L)ₐ-(CH₂)_{b}-(T)_{c}-B wherein a, b, c, L, T and B are as defined above;
R₁₇ is H, phenyl, benzyl or C₁₋₄alkyl.

Suitably, R₁ is C₈₋₁₃alkyl, C₇₋₁₂alkoxy, C₇₋₁₂alkylthio, C₁₀₋₁₂1-alkynyl, phenyl C₄₋₁₀alkyl, phenylC₃₋₉alkoxy, phenylthioC₃₋₉alkyl, 10-undecynyloxy or 11-dodecynyloxy.

More suitably, R₁ is C₁₁₋₁₃alkyl, C₁₀₋₁₂alkoxy or phenylC₇₋₉alkyl;

Suitably, R₂ is bromo, chloro, methyl, trifluoromethyl, hydroxy, methoxy, or nitro, or when R₁ is hydrogen, R₂ is C₈₋₁₃ alkyl, C₇₋₁₂alkoxy, C₇₋₁₂alkylthio, C₁₀₋₁₂1-alkynyl, phenylC₄₋₁₀alkyl, phenylC₃₋₉alkoxy, phenylthioC₃₋₉alkyl, 10-undecynyloxy or 11-dodecynyloxy.

Preferably, A and R₂ are H.

Preferably, R₁ is phenyloctyl.

Preferably, R₁₇ is H.

A particular compound of this invention is:
(2R-trans)-3-[2-(8-phenyloctyl)phenyl]oxirane carboxamide.

One process for preparing a compound of the formula (IV) comprises reacting a compound of the formula (V): 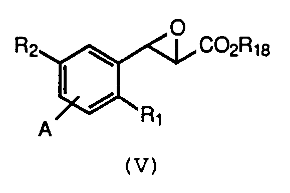
wherein:
R₁, R₂ and A are as hereinbefore defined for formula (IV) and R₁₈ is C₁₋₄alkyl, phenyl, naphthyl, substituted phenyl, or substituted naphthyl, where the substituents may be one or two halogen, C₁₋₄alkyl, C₁₋₄alkoxy or trifluoromethyl groups, with a compound of the formula:

R₁₇NH₂

wherein R₁₇ is H, phenyl, benzyl or C₁₋₄alkyl. This process is depicted in Scheme (I). 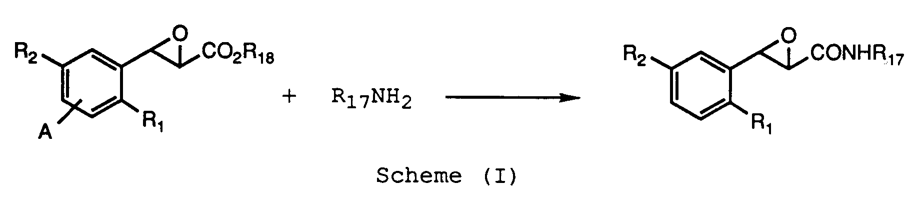
The esters of formula (IV) are conveniently prepared by a Darzen's condensation of an α-halo-ester, such as methyl chloroacetate or α-bromo-t-butyl acetate, with a benzaldehyde of formula (VI), in the presence of a suitable base, such as sodium methoxide. 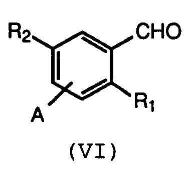
These α-epoxy esters are reacted with ammonia, a C₁₋₄alkyl amine, benzylamine or aniline in a suitable solvent to provide the carboxamide in the usual fashion. Examples of suitable solvents are alcohols, such as methanol or ethanol, and hydrocarbon solvents, such as acetone, tetrahydrofuran or toluene. Typically the ester is stirred in solution saturated with ammonia or containing an excess of an amine, such as ethylamine, benzylamine or aniline, at a temperature of from about room temperature to the reflux temperature of the solvent. Addition of ammonium hydroxide is suitable for saturation of the solvent with ammonia.

If a non-racemic epoxy-ester is used in this process, the resultant amide is also non-racemic. Thus, individually both enantiomers of the compounds of formula (IV) are within the scope of this invention. One method for producing a nonracemic α-epoxy ester of formula (V), wherein R₁₈ is phenyl, naphthyl or substituted phenyl or substituted naphthyl, is disclosed in U.S. patent application 07/366,059. Accordingly, a benzaldehyde of formula (VI), wherein R₁, R₂ and A are as hereinbefore defined for formula (IV), is condensed with an acetophenone or acetonaphthone of formula (VII), wherein R₁₈ is phenyl, naphthyl or a substituted phenyl or substituted naphthyl, in the presence of a suitable base to produce an α,β-unsaturated ketone of formula (VIII). 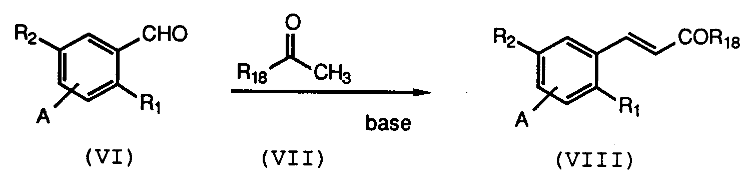
Suitably, this reaction is conducted in an alcohol, such as methanol or ethanol with a base, such as a sodium or potassium alkoxide. Enantioselective epoxidation is performed by stirring the α,β-unsaturated ketone with a polyamino acid, such as poly-L-leucine or poly-L-alanine in the presence of a suitable base, such as lithium, sodium or potassium hydroxide, and an oxidant, such as hydrogen peroxide, in a two phase solvent mixture of water and a water immiscible organic solvent, such as carbon tetrachloride, hexane or toluene, to yield an α-epoxy ketone of formula (IX). 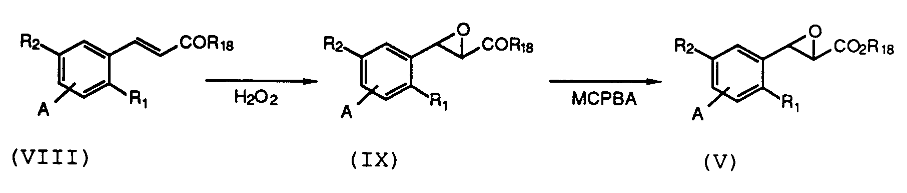
For the compounds of this invention, poly-L-amino acids produce 2R isomers, whereas poly-D-amino acids produce 2S isomers. Baeyer-Villiger rearrangement of the nonracemic epoxide is accomplished by treating the α-epoxy ketone with a peracid, such as 3-chloroperoxybenzoic acid, to produce the α-epoxy ester of formula (V), wherein R₁₈ is phenyl, naphthyl or a substituted phenyl or substituted naphthyl as hereinbefore described. Subsequent treatment of this ester with a suitable amine, or ammonia, yields the nonracemic compounds of formula (IV), as hereinbefore described. As will be apparent to one skilled in the art, compounds which possess functionalities which are incompatible with the oxidative conditions of this process, such as sulfide, amino and acetylenic moieties, are not amenable to nonracemic synthesis in this manner.

Alternatively, other methods common to the art for producing α-epoxy amides are also useful. For instance, the carboxamide of formula (IV) may be produced by amination of a glycidic acid. In another embodiment, compounds of formula (IV) may be prepared by reacting a compound of formula (VI), as hereinbefore defined, with a compound of the formula:

X′-CH₂CONHR₁₇

wherein R₁₇ is as defined for formula (IV) and X′ is chloro, bromo or iodo. Typically, a benzaldehyde of formula (VI) is reacted with a suitable α-halo acetamide or substituted α-halo acetamide, in a suitable solvent, such as a lower alkyl alcohol or methylene chloride, and a base, such as an alkoxide, to produce the desired carboxamide directly. This process is depicted in Scheme (II). 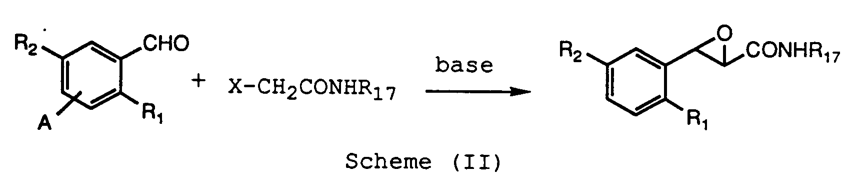
For example, bromoacetamide or N-phenylchloroacetamide may be reacted with a benzaldehyde of formula (VI), and sodium methoxide in methylene chloride or methanol to yield a carboxamide of formula (IV).

The carboxamides of formula (IV) are particularly useful intermediates for the preparation of leukotriene antagonists of formula (I). Using conditions analogous to those reported for preparing the compounds of formula (I) from the esters of formula (II), such as those reported by Gleason et al., J.Med. Chem., 30, 959(1987), the amide is stirred in an alcoholic solution with a base, such as triethylamine and a suitable mercaptan, such as methyl 3-mercaptopropionate. Such a process results in a 1:1 mixture of regioisomers, resulting from nucleophilic opening of the epoxide at both the α and β positions. However, a particular advantage of the compounds of formula (IV), is their use in a process which permits regioselective synthesis of the compounds of formula (I). This may be achieved by using a titanium mediated reaction to promote nucleophilic addition to the epoxide intermediate in a regioselective manner. Accordingly, an α-epoxy amide of formula (IV) is treated with an excess of titanium isopropoxide and an appropriate thiolate, such as R₃-S-M, wherein R₃ is as defined for formula (I) and M is lithium, sodium or potassium, in a suitable solvent to yield the α-hydroxy, β-sulfido amide with a selectivity of generally >20:1 compared to the regioisomer. Aprotic solvents are generally suitable. Ethers or halocarbon solvents, such as tetrahydrofuran, dioxane, diethyl ether, methylene chloride and carbon tetrachloride are especially suitable. Subsequent hydrolysis to yield a carboxylic acid and optionally reforming the ester provides an overall conversion from an α-epoxy ester of formula (II) or (V) to the pharmaceutically active products of formula (I).

Thus, this invention is a process for preparing a compound of the formula (I), as hereinbefore defined, which comprises reacting a compound of the formula (IV) with titanium isopropoxide and a compound of the formula:

R₃-S-M

wherein R₃ is as defined for formula (I) and M is lithium, sodium or potassium; and hydrolyzing the product thereof. This process is depicted in Scheme (III). 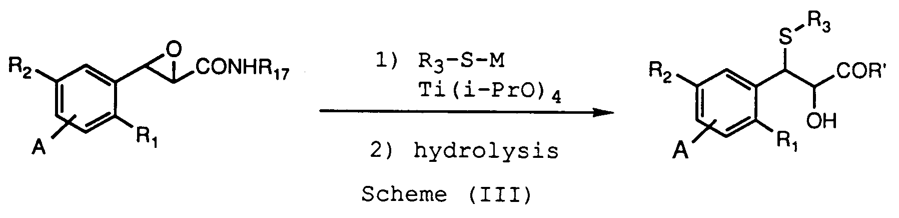
In a typical process, one equivalent of an α-epoxy amide is reacted under anhydrous conditions with 2.5 equivalents of titanium isopropoxide at 0°C in a solvent such as THF. This mixture is further treated with an alkali metal salt of an appropriate mercaptan, such as methyl 3-mercaptopropionate or methyl-p-mercaptobenzoate, and stirred at 0°C. Aqueous workup followed by extractive isolation provides the desired product. Acid derivatives of the compounds of formula (I) are provided by hydrolysis of the amide, which may be accomplished with a suitable mineral acid by well known methods. Alternatively an alcoholic solution of a mineral acid, such as HCl, may be used to prepare simple alkyl esters, which may be converted by basic hydrolysis, such as with an alkali metal hydroxide in aqueous alcohol, to the carboxylic acid. Esters are produced in any convenient fashion for converting carboxamides or carboxylic acids to esters. For instance, methyl esters are conveniently prepared from the carboxamide with methanol and a mineral acid, as previouly described, or from the carboxylic acid by treatment with diazomethane. A preferred compound prepared by this process is (R*,S*) 2-hydroxy-3-[2-(carboxy)ethylthio]-3-[2-(8-phenyloctyl)phenyl]-propanoic acid.

Use of the compounds and process of this invention provide a new method for producing leukotriene antagonists of formula (I). This process results in higher synthetic yields in the epoxide opening reaction resulting from elimination of the undesired reaction of α addition of the mercaptan to the epoxide moiety. In addition, product purification is simplified due to the absence of the regioisomer, which was a major by-product of the chemical process. Product purification and isolation are further facilitated by the tendency of the amides of formula (IV) to be crystalline. Finally, the primary amides of this invention are more economical to prepare and easier to hydrolyze to carboxylic acids than secondary amides previously used in regioselective epoxide openings.

The Examples which follow illustrate the manner of making and using this invention. The scope of this invention is not limited by these examples and the many variations which will be readily apparent to one skilled in the art are intended to be encompassed by this disclosure.

Conventions common in the chemical arts are used in the Examples. All melting points are uncorrected. In the notations to the NMR spectra, the following abbreviations are used: s, singlet; d, doublet; t, triplet; q, quartet; m, multiplet; br, broad. In notations to the IR spectra, the following abbreviations are used: s, strong; sh, shoulder.

### Example 1

### Preparation of (2S-trans)-3-[2-(8-phenyloctyl)phenyl]oxiranecarboxamide.

(2S-trans)-2-naphthalenyl 3-[2-(8- phenyloctyl)phenyl]oxiranecarboxylate (2.03 g, 4.2 mmol), in methanol (21 mL), was cooled to 5°C and a solution of ammonia saturated methanol (20 mL) was added dropwise. After stirring for 5 h at 10-15°C, the solution was evaporated. The residue was dissolved in toluene and evaporated to a solid. The solid was dissolved in toluene and washed with water, NaOH (10 % aq.), water, and finally brine. After drying (MgSO₄) and evaporation, the residue was dissolved in boiling hexane:CH₂Cl₂, filtered and allowed to stand at 20°C for 4 h. The solid was collected and dried (0.1 mm Hg, 25°C) to yield the titled oxiranecarboxamide (1.11 g, 76%): mp 80-82°C; [α]_{D} -8.3° (c 1, CH₂Cl₂); HPLC RT 5.48 min (2S-enantiomer), 9.99 min (2R-enantiomer) (Bakerbond Chiracel® OC; 25 cm x 4.6 mm; hexane:isopropanol, 1:1; 1.0 mL/min; UV detection at 211 nm); HPLC indicated > 99% e.e., the 2S enantiomer predominating. Anal. Calcd for C₂₃H₂₉O₂N: C, 78.59; H, 8.32; N, 3.99; Found: C, 78.35; H, 8.34; N, 3.90.

### Example 2

### Preparation of (2R-trans)-3-[2-(8-phenyloctyl)phenyl]oxiranecarboxamide.

To a vigorously stirred solution of (2R-trans)-2-naphthalenyl 3-[2-(8-phenyloctyl)phenyl]oxiranecarboxylate (486 g, 1.016 mol) in acetone (5.0 L) was added ammonium hydroxide (28-30% aq., 1.0 L), and the mixture was refluxed for 20 min. The reaction was judged to be complete by HPLC and was concentrated in vacuo to 1/3 of the original volume and toluene (10 L) was added. The solvents were distilled until the head temperature rose to 105°C. The reaction was cooled to 25°C, and washed sequentially with NaOH (10% aq., 3 x 5 L), water, and brine. The mixture was concentrated to 2.5 L and treated with n-hexane (7.5 L). A precipitate formed and the solution was kept at 0°C for 12 h. The product was collected, washed with cold hexane (2 x 1 L) and dried in vacuo (0.1 mm Hg) to give a white solid (308.5 g, 86.5%): mp 81-82°C; [α]_{D} +13.2° (c 1, CH₂Cl₂), [α]₅₄₆ +16.1°; IR (nujol) 3380, 1668(s), 1620(sh), 1283, 1088, 897, 750, 700 cm⁻¹; ¹H NMR (CDCl₃, 400 MHz) δ 7.14-7.28 (m, 9H), 6.19 (br, s, 1 H), 5.76 (br, s, 1 H), 4.11 (d, 1 H, J=1.90 Hz, oxirane proton), 3.37 (d, 1 H, J=1.68 Hz, oxirane proton), 2.65 (m, 2 H), 2.59 (t, 2 H, J=7.70 Hz), 1.60 (m, 4 H), 1.32 (s, 8 H); ¹³C NMR (CDCl₃, 100 MHz) δ 170.24, 142.88, 141.33, 132.67, 129.21, 128.54, 128.38, 128.19, 126.24, 125.53, 124.19, 58.07, 56.93, 35.94, 32.57, 31.46, 30.89, 29.43, 29.38, 29.34, 29.26; HPLC RT 2.5 min, (epoxyamide product), 6.9 min(epoxy ester starting material),(Waters µ-Bondapak® C-18; 30 cm x 3.9 mm; CH₃CN:water, 9:1; 2 mL/min; UV detection at 211 nm); HPLC (chiral) RT 9.9 min (2R-enantiomer), 5.5 min (2S-enantiomer) (Bakerbond Chiracel® OC ; 25 cm x 4.6 mm; n-hexane:isopropanol, 1.1 mL/min; UV detection at 211 nm); HPLC indicated >99.5% e.e., the 2R enantiomer predominating. Anal. Calcd for C₂₃H₂₉O₂N: C, 78.59; H, 8.32; N, 3.99. Found: C, 78.63; H, 8.87; N, 3.98.

### Example 3

### Preparation of (R*,S*) 3-[2-(undecylthyio)phenyl]oxiranecarboxamide.

To a solution of (trans)-methyl 3-[2-(undecylthio)phenyl]oxiranecarboxylate (1 g) in acetone (5 mL) is added ammonium hydroxide (1 mL, 30%). The reaction is refluxed for 1 h, cooled and diluted with toluene (50 mL). The solvent is evaporated to approximately 50% of its original volume and washed with water (2x) and brine (1x). The solution is dried briefly over MgSO₄, filtered and the solvent is evaporated to yield the titled product.

### Example 4

### Preparation of (R*,S*) 3-[2-(1-dodecynyl)phenyl]oxiranecarboxamide.

To a solution of (trans)-methyl 3-[2-(1-dodecynyl)phenyl]oxirane carboxylate (1 g, 3.31 mmol) in methanol (10 mL) is added a solution of ammonia saturated methanol (10 mL). After stirring for 14 h at room temperature, the methanol is evaporated and the residue is redissolved in methylene chloride. The organic phase is washed with water (2x), dried over MgSO₄, filtered and the solvent is evaporated to yield the titled product.

### Example 5

### Preparation of (R*,S*) N-benzyl-3-[2-(1-nonenyl)phenyl]oxiranecarboxamide.

To a solution of (trans)-methyl 3-[2-(1-nonenyl)phenyl]oxirane carboxylate (1 g, 3.31 mmol) in absolute ethanol (10 mL), benzylamine (0.41 g, 3.81 mmol) is added. The reaction is warmed to reflux for 2 1/2 h and allowed to stir at room temperature overnight. The reaction mixture is diluted with toluene (80 mL) and evaporated on a rotary evaporator to a volume of 10 mL. The carboxamide is precipitated by the addition of hexane and the solvent is decanted. Flash chromatography (silica gel, ethyl acetate:hexane:0.5% triethylamine) provides the titled compound.

### Example 6

### Preparation of (R*,S*) N-ethyl-3-[2-(4-phenoxybutyl)-5-(amino)phenyl]oxiranecarboxamide.

To a solution of (trans)-methyl 3-[2-(4-phenoxybutyl)-5-(amino)phenyl]oxirane carboxylate (1 g) in methanol (6 mL) is added ethylamine (3 mL). The reaction mixture is sealed tightly and stirred at room temperature for 24 h. The solvent and excess ethylamine are evaporated and the residue is dissolved in diethyl ether, washed with water (2x) and dried briefly over MgSO₄. Filtration and evaporation of the solvent yields the titled compound.

### Example 7

### Preparation of (R*,S*) N-phenyl-3-[2-(8-phenyloctyl)phenyl]oxiranecarboxamide.

2-(8-phenyloctyl)benzaldehyde (1.5 g, 6.4 mmol) is dissolved in methylene chloride (25 mL) and cooled to 0°C. N-phenylchloroacetamide (1.35 g, 8.0 mmol) is added, followed by sodium methoxide (1.75 mL, 7.68 mmol, 25 wt % in methanol) which is added dropwise. After stirring at 0°C for 1 h, the reaction is allowed to stir at room temperature for an additional 3 h. The mixture is poured into ice water (200 mL) and extracted with ethyl acetate (2 X 100 mL). The combined organic extracts are washed with water (100 mL) and brine (100 mL) and dried over MgSO₄. Filtration and evaporation of the solvent yields the titled compound, which is recrystallized from absolute ethanol.

### Example 8

### Preparation of (R*,S*) 3-[3-(9-decynyl)phenyl]oxiranecarboxamide.

3-(9-decynyl)benzaldehyde (1.0 g, 4.7 mmol) and bromoacetamide (0.676 g, 4.9 mmol) are dissolved in methanol (20 mL). Sodium methoxide(4.9 mmol, 25 wt % in methanol) is added dropwise and the reaction is stirred at room temperature for 4 h. Ammonium chloride is added (2.5 mL, 10 % aq.) and the solvent is reduced in vacuo to 1/3 of the original volume. The mixture is diluted with water (40 mL) and methylene chloride (20 mL). The organic layer is separated and the aqueous is extracted with additional methylene chloride (20 mL). The combined organic extracts are washed with water and dried over MgSO₄. Filtration and evaporation of the solvent yields the titled compound.

### Example 9

Substituting a 2-naphthalenyl α-epoxy ester, listed below:
(2R-trans)-(2-naphthalenyl) 3-[(2-dodecyl)phenyl]oxiranecarboxylate;
(2R-trans)-(2-naphthalenyl) 3-[2-(12,12,12-trifluorododecyl)phenyl]oxiranecarboxylate;
(2R-trans)-(2-naphthalenyl) 3-[2-(undecyloxy)phenyl]oxiranecarboxylate;
(2R-trans)-(2-naphthalenyl) 3-[2-(8-phenyloctyl)-5-(trifluoromethyl)phenyl]oxiranecarboxylate;
(2R-trans)-(2-naphthalenyl) 3-[2-[(8-cyclohexyl)octyl]phenyl]oxiranecarboxylate;
(2R-trans)-(2-naphthalenyl) 3-[3-(6-phenylhexyl)phenyl]oxiranecarboxylate;
(2R-trans)-(2-naphthalenyl) 3-[5-nitro-2-(undecyloxy)phenyl]oxiranecarboxylate;
(2R-trans)-(2-naphthalenyl) 3-[2-[8-(2-furyl)octyl]phenyl]oxiranecarboxylate;
(2R-trans)-(2-naphthalenyl) 3-[3-(nonyloxy)phenyl]oxiranecarboxylate;
(2R-trans)-(2-naphthalenyl) 3-[2-[5-bromo-6-(4-methylphenyl)hexyl]phenyl]oxiranecarboxylate;
(2R-trans)-(2-naphthalenyl) 3-[2-[7-(4-chlorophenyl)heptyl]-5-(methyl)phenyl]oxiranecarboxylate;
(2R-trans)-(2-naphthalenyl) 3-[2-[8-(4-methoxyphenyl)octyl]phenyl]oxiranecarboxylate;
(2R-trans)-(2-naphthalenyl) 3-[2-[10-(2,4-dimethylphenyl)decyl]-3-(nitro)phenyl]oxiranecarboxylate;
(2R-trans)-(2-naphthalenyl) 3-[2-[8-(2-thienyl)octyl]-3-(trifluoromethyl)phenyl]oxiranecarboxylate; and
(2R-trans)-(2-naphthalenyl) 3-[2-[8-[(3-trifluoromethyl)phenyl]octyl]phenyl]oxiranecarboxylate;
for (2S-trans)-2-naphthalenyl 3-[2-(8- phenyloctyl)phenyl] oxiranecarboxylate in the procedure of Example 1, the following α-epoxyamides are prepared:
a.) (2R-trans) 3-[(2-dodecyl)phenyl]oxiranecarboxamide;
b.) (2R-trans) 3-[2-(12,12,12-trifluorododecyl)phenyl]oxiranecarboxamide;
c.) (2R-trans) 3-[2-(undecyloxy)phenyl]oxiranecarboxamide;
d.) (2R-trans) 3-[2-(8-phenyloctyl)-5-(trifluoromethyl)phenyl]oxiranecarboxamide;
e.) (2R-trans) 3-[2-[(8-cyclohexyl)octyl]phenyl]oxiranecarboxamide;
f.) (2R-trans) 3-[3-(6-phenylhexyl)phenyl]oxiranecarboxamide;
g.) (2R-trans) 3-[5-nitro-2-(undecyloxy)phenyl]oxiranecarboxamide;
h.) (2R-trans) 3-[2-[8-(2-furyl)octyl]phenyl]oxiranecarboxamide;
i.) (2R-trans) 3-[3-(nonyloxy)phenyl]oxiranecarboxamide;
j.) (2R-trans) 3-[2-[5-bromo-6-(4-methylphenyl)hexyl]phenyl]oxiranecarboxamide;
k.) (2R-trans) 3-[2-[7-(4-chlorophenyl)heptyl]-5-(methyl)phenyl]oxiranecarboxamide;
l.) (2R-trans) 3-[2-[8-(4-methoxyphenyl)octyl]phenyl]oxiranecarboxamide;
m.) (2R-trans) 3-[2-[10-(2,4-dimethylphenyl)decyl]-3-(nitro)phenyl]oxiranecarboxamide;
n.) (2R-trans) 3-[2-[8-(2-thienyl)octyl]-3-(trifluoromethyl)phenyl]oxiranecarboxamide; and
o.) (2R-trans) 3-[2-[8-[(3-trifluoromethyl)phenyl]octyl]phenyl]oxiranecarboxamide.

### Example 10

### Preparation of (R*,S*) 2-hydroxy-3-[2-(carboxy)ethylthio]-3-[2-(8-phenyloctyl)phenyl]propanoic acid.

### a) Preparation of (R*,S*) N-phenyl-2-hydroxy-3-[2-(methoxycarbonyl)ethylthio]-3-[2-(8-phenyloctyl)phenyl]propanamide.

To a cooled (-5°C) mixture of sodium hydride (60% in mineral oil, .302 g, 6.3 mmol) and tetrahydrofuran (10 mL) was added dropwise methyl 3-mercaptopropionate (.712 mL, 6.3 mmol). After the addition was complete, stirring was continued at 0°C for 10 min. To a cooled (-5°C) solution of (R*,S*) N-phenyl-3-[2-(8-phenyloctyl)phenyl]oxiranecarboxamide (1.28 g, 3.0 mmol), the compound of Example 7, in tetrahydrofuran (10 mL) was added titanium isopropoxide (1.34 mL, 4.5 mmol) over a period of several min. After stirring at -5°C for a period of 15 min, the solution was added to the cold (0°C) thiolate/tetrahydrofuran mixture over a period of 10 min. After stirring at 0°C for a period of 75 min, the solution was diluted with ethyl ether (10 mL) followed by the addition of aqueous sulfuric acid (10%, 10 mL) over a period of 5-10 min. After stirring at ambient temperature for a period of 45 min, the mixture was transferred to a separatory funnel with additional ethyl ether (75 mL) and the layers separated. The organic phase was washed with aqueous sodium chloride (25%, 3 x 75 mL), dried (MgSO₄) and concentrated in vacuo. The resulting oil was triturated with hexanes (20 mL) and ethyl ether (1 mL). After several min, additional hexanes (10 mL) was added and stirring continued for another 5 min. The mixture was filtered, and the solid product washed with hexanes and dried in vacuo to afford 1.35 g (82.3%) of the desired product as a white solid: ¹H NMR (CDCl₃, 400 MHz) δ 8.11 (s, 1 H), 7.57-7.60 (m, 1 H), 7.03-7.29 (m, 13 H), 4.81 (d, 1 H, J=4.16 Hz), 4.66 (d, 1 H, J=4.17 Hz), 3.72 (s, 3 H), 2.72-2.96 (m, 4 H), 2.54-2.70 (m, 4 H), 1.62 (m, 4 H), 1.26 (bs, 8 H).

### b) Preparation of (R*,S*) 2-hydroxy-3-[2-(carboxy)ethylthio]-3-[2-(8-phenyloctyl)phenyl]-propanoic acid

To a solution of the compound of Example 10a (.274 g, 0.5 mmol), methylene chloride (4 mL), triethylamine (.140 mL, 1.0 mmol) and dimethylaminopyridine (.122 g, 1.0 mmol) at ambient temperature was added di-t-butyl-dicarbonate (.474 mL, 2.0 mmol). After stirring at ambient temperature for a period of 3 h, the reaction was diluted with ethyl acetate (50 mL), transferred to a separatory funnel, washed with aqueous hydrochloric acid (10%, 2 x 50 mL), dried (Na₂SO₄), filtered, and concentrated in vacuo. The resulting oil was purified by flash chromatography (silica gel, 20% ethyl acetate/petroleum ether) affording the desired intermediate (.354 g, 94.6%). To a solution of the purified intermediate (.354 g, .47 mmol) in tetrahydrofuran (6 mL) at ambient temperature was added aqueous lithium hydroxide (1N, 2 mL). After stirring for a period of 9 h, the reaction was diluted with ethyl acetate (50 mL), transferred to a separatory funnel, washed sequentially with aqueous hydrochloric acid (10%, 2 x 50 mL) and aqueous sodium chloride (25%, 50 mL), dried (Na₂SO₄), filtered and concentrated in vacuo. The resulting oil was redissolved in methylene chloride (5 mL) and cooled to 0°C. To this was added trifluoroacetic acid (1 mL) and the mixture stirred at 0°C for a period of 15 min followed by 15 min at ambient temperature. The solution was diluted with ethyl acetate (50 mL), transferred to a separatory funnel, washed sequentially with aqueous hydrochloric acid (10%, 2 x 50 mL) and aqueous sodium chloride (25%, 50 mL), dried (Na₂SO₄), filtered and concentrated in vacuo to afford the desired product as a viscous oil. Trituration of this material with hexane:diethyl ether yielded the product as a white solid: ¹H NMR (CDCl₃, 400 MHz) δ 7.57 (d, 1 H, J=7.6 Hz), 7.30-7.09 (m, 8 H), 6.43 (s, 1 H), 5.42 (s, 1 H), 4.70 (d, 1 H, J=4.2 Hz), 4.54 (d, 1 H, J=4.3 Hz), 3.70 (s, 3 H), 2.91-2.74 (m, 4 H), 2.68-2.53 (m, 4 H), 1.62-1.60 (m, 4 H), 1.34 (bs, 8 H); Anal. Calcd for C₂₇H₃₇NO₄S: C, 68.75; H, 7.91; N, 2.97; S, 6.80. Found: C, 68.49; H, 8.05; N, 2.97; S, 6.84.

### Example 11

### Preparation of (2S,3R) 2-hydroxy-3-[2-(carboxy)ethylthio]-3-[2-(8-phenyloctyl)phenyl]propanoic acid.

### a) Preparation of (2S,3R) 2-hydroxy-3-[2-(methoxycarbonyl)ethylthio]-3-[2-(8-phenyloctyl)phenyl]propanamide.

To a cooled (-10°C) mixture of sodium hydride (60%, 9.6 g, .238 mol) and methylene chloride (280 mL) was added methyl 3-mercaptopropionate (27.0 mL, .238 mol) over a period of 5 min. After the addition was complete, stirring was continued at -5 - 0°C for 30 min. While maintaining the temperature at -5 - 0°C, a solution of the compound of Example 2 (40.0 g, .110 mol) and titanium isopropoxide (84.82 mL, .284 mol) in methylene chloride (120 mL) at ambient temperature was added over a period of 20 min. After stirring at -5 - 0°C for a period of 90 min, the solution was diluted with ethyl acetate (400 mL) followed by the addition of aqueous sulfuric acid (10%, 400 mL) over a period of 10 min. After stirring at ambient temperature for a period of 90 min, the mixture was transferred to a separatory funnel and the phases separated. The aqueous phase was extracted with ethyl acetate (2 X 200 mL) and the combined organic phases were concentrated in vacuo to afford 80.0 g of a viscous yellow oil (61.9% purity assay, 95.4% corrected yield). Typically, the crude product was used immediately in the next step without further purification. However, a purified sample of the title compound could be prepared by the following method. The crude oil was first slurried in hexanes (2 x 100 mL), and the hexanes removed by decantation. The residue was then purified further by flash chromatography (silica gel, 1:1 ethyl acetate:petroleum ether). After concentrating in vacuo the desired fractions, the residual oil was triturated with hexanes (100 mL). The mixture was then filtered, and the solid washed with hexanes and dried in vacuo at ambient temperature to afford the desired product as a white powder: mp 41.5-42.5°C; IR (KBr) 3600-2800, 3477, 1728, 1668, 1604, 1571, 1368, 1223, 1204, 1157, 751, 700 cm⁻¹; ¹H NMR (CDCl₃, 400 MHz) δ 7.57 (d, 1 H, J=7.6 Hz), 7.30-7.09 (m, 8 H), 6.43 (s, 1 H), 5.42 (s, 1 H), 4.70 (d, 1 H, J=4.2 Hz), 4.54 (d, 1 H, J=4.3 Hz), 3.70 (s, 3 H), 2.91-2.74 (m, 4 H), 2.68-2.53 (m, 4 H), 1.62-1.60 (m, 4 H), 1.34 (bs, 8 H); Anal. Calcd for C₂₇H₃₇NO₄S: C, 68.75; H, 7.91; N, 2.97; S, 6.80. Found: C, 68.49; H, 8.05; N, 2.97; S, 6.84.

### b) Preparation of (2S,3R) 2-hydroxy-3-[2-(carboxy)ethylthio]-3-[2-(8-phenyloctyl)phenyl]-propanoic acid.

To a cooled (10°C) solution of the compound of Example 11a (61.9%, 80.0 g, .105 mol) in anhydrous methanol (500 mL) was added 2.5 N sodium hydroxide (155 mL, .387 mol) over a period of 10 min, allowing the reaction temperature to rise to approximately 22°C. After stirring at ambient temperature for a period of 16 h, the reaction mixture was concentrated in vacuo to remove the methanol. The aqueous concentrate was diluted with additional deionized water (400 mL), transferred to a separatory funnel and washed with ethyl acetate (1 x 400 mL). The aqueous phase was then combined with fresh ethyl acetate (400 mL) and saturated with the addition of solid sodium chloride. The layers were separated and the aqueous phase was back extracted with ethyl acetate (1 x 200 mL). The combined organic extracts were then washed with aqueous sodium chloride (20%, 1 x 100 mL) and concentrated in vacuo to afford 85.0 g of the desired intermediate as a viscous oil. The crude intermediate was redissolved in methanol (1000 mL), then treated with aqueous hydrochloric acid (25%, 480 mL). The mixture was heated at reflux for a period of 20 h, then carefully cooled to 5-10°C. To this was added solid sodium hydroxide (3 x 50 g) portionwise, allowing the temperature to cool to 10°C between additions. After the final addition, the solution was allowed to warm to ambient temperature and stirred for a period of 2 h. The solution was diluted with deionized water (500 mL), concentrated in vacuo to remove the methanol and extracted with ethyl acetate (4 x 500 mL, discarded). The aqueous phase was treated with ethyl acetate (500 mL) and the pH adjusted to 2.0 with concentrated hydrochloric acid. This mixture was transferred to a separatory funnel and the layers were separated. The aqueous phase was extracted with ethyl acetate (1 x 200 mL). The combined ethyl acetate extracts were washed with aqueous sodium chloride (20%, 250 mL) then concentrated in vacuo to afford 55.0 g of the desired product as as viscous oil: ¹H NMR (CDCl₃, 400 MHz) δ 7.55 (d, 1 H, J=7.55 Hz), 7.15-7.28 (m, 8 H), 4.62 (d, 1 H, J=5.65 Hz), 4.56 (d, 1 H, J=5.53 Hz), 2.81-2.68 (m, 4 H), 2.57-2.64 (m, 4 H), 1.60-1.57 (m, 4 H), 1.32 (bs, 8 H).

### Example 12

### Preparation of (2S,3R)-methyl 3-[(4-carbomethoxy)phenylthio]-2-hydroxy-3-[2-(8-phenyloctyl)phenyl]propionate.

### a) (2S,3R) 2-hydroxy-3-[(4-carbomethoxy)-phenylthio]-3-[2-(8-phenyloctyl)phenyl]propanamide.

A solution of the compound of Example 2 (4.9 g, 14 mmol) in THF (100 mL) at 0° was treated with titanium isopropoxide (10.4 mL, 35 mmol), and stirred 30 minutes. This was then treated with a solution of methyl-p-mercaptobenzoate (3.5 g, 21 mmol) in THF (5O mL) which had been treated with NaH (840 mg, 21 mmol, 60% in mineral oil). The combined solution was stirred at 0° for 2 h, poured into H₂SO₄ (400 mL, 10% aq.), and extracted with Et₂O. The extracts were washed with H₂O and aqueous Na₂CO₃, dried and the the solvent removed. The residue was purified by flash chromatography (silica gel, 1:10 methanol:ethyl acetate) to afford a quantitative recovery of the titled product: ¹H NMR (CDCl₃, 90 MHz) δ 7.93(d, 2H), 7.69(m, 1H), 7.46(d, 2H), 6.98-7.40(m, 8H), 6.30(d, 1H), 5.62(d, 1H), 5.22(d, 1H), 4.42(t, 1H), 3.88(s, 3H), 2.40-3.02(m, 5H), 1.09-1.82(m, 12H).

### b) (2S,3R)-methyl 3-[(4-carbomethoxy)-phenylthio]-2-hydroxy-3-[2-(8-phenyloctyl)phenyl]-propionate.

A solution of of the compound of Example 12a (7.6 g), in a mixture of MeOH (180 mL), conc. HCl (15 mL) and H₂O (10 mL), was refluxed 16 h. The MeOH was removed, and the residue extracted with Et₂O. The extracts were washed with H₂O, dried, and the solvent removed. The residue was purified by flash chromatography (silica gel, 2:3 ethyl acetate:hexane) to provide the titled product (6.4 gm, 89%). ¹H NMR(CDCl₃, 90 MHz) δ 7.94(d, 2H), 7.62(m, 1H), 7.40(d, 2H), 6.97-7.27(m, 8H), 4.95(d, 1H), 4.54(t, 1H), 3.82(s, 3H), 3.52(s, 3H), 3.13(d, 1H), 2.37-2.80(m, 4H), 1.03-1.87(m, 12H).

Based upon this disclosure many variations and modifications of these procedures will be appparent to those skilled in the chemical arts. Such modifications and variations are intended to be comprehended within the scope of this invention and encompassed by the claims which follow.

## Claims (Claims for the following Contracting State(s): AT, BE, CH, DE, DK, FR, GB, GR, IT, LI, LU, NL, SE)

1. A compound of the formula: 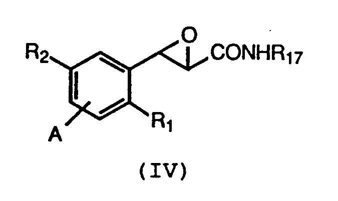 wherein:
R₁ is (L)ₐ-(CH₂)_{b}-(T)_{c}-B;
a is 0 or 1;
b is 3 to 14;
c is 0 or 1;
L and T are independently sulfur, oxygen, CH=CH, C≡C, or CH₂;
B is H, C₁₋₄alkyl, ethynyl, trifluoromethyl, isopropenyl, furanyl, thienyl, cyclohexyl or phenyl optionally monosubstituted with Br, Cl, CF₃, C₁₋₄alkoxy, C₁₋₄alkyl, methylthio or trifluoromethylthio;
R₂ and A are independently selected from H, CF₃, C₁₋₄alkyl, C₁₋₄alkoxy, F, Cl, Br, I, OH, NO₂ or NH₂; or, when R₁ and A are H, R₂ is (L)ₐ-(CH₂)_{b}-(T)_{c}-B wherein a, b, c, L, T and B are as defined above; and
R₁₇ is H, phenyl, benzyl or C₁₋₄alkyl.

2. A compound according to claim 1 in which R₁ is C₈₋₁₃alkyl, C₇₋₁₂alkoxy, C₇₋₁₂alkylthio, C₁₀₋₁₂1-alkynyl, phenylC₄₋₁₀alkyl, phenylC₃₋₉alkoxy, phenylthioC₃₋₉alkyl, 10-undecynyloxy or 11-dodecynyloxy.

3. A compound according to claim 1 in which R₂ is bromo, chloro, methyl, trifluoromethyl, hydroxy, methoxy, or nitro, or when R₁ and A are hydrogen, R₂ is C₈₋₁₃ alkyl, C₇₋₁₂alkoxy, C₇₋₁₂alkylthio, C₁₀₋₁₂1-alkynyl, phenylC₄₋₁₀alkyl, phenylC₃₋₉alkoxy, phenylthioC₃₋₉alkyl, 10-undecynyloxy or 11-dodecynyloxy.

4. A compound according to claim 1 in which A and R₂ are both H.

5. A compound according to claim 1 in which R₁₇ is H.

6. A compound according to claim 2 in which R₁ is C₁₁₋₁₃alkyl, C₁₀₋₁₂alkoxy or phenylC₇₋₉alkyl

7. A compound according to claim 6 in which R₁ is phenyloctyl.

8. A compound according to claim 7 which is (2R-trans)-3-[2-(8-phenyloctyl)phenyl]oxiranecarboxamide.

9. A process for preparing a compound of the formula: 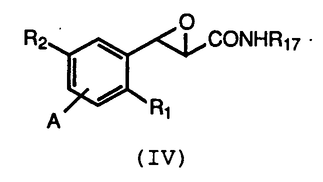 wherein:
R₁ is (L)ₐ-(CH₂)_{b}-(T)_{c}-B;
a is 0 or 1;
b is 3 to 14;
c is 0 or 1;
L and T are independently sulfur, oxygen, CH=CH, C≡C, or CH₂;
B is H, C₁₋₄alkyl, ethynyl, trifluoromethyl, isopropenyl, furanyl, thienyl, cyclohexyl or phenyl optionally monosubstituted with Br, Cl, CF₃, C₁₋₄alkoxy, C₁₋₄alkyl, methylthio or trifluoromethylthio;
R₂ and A are independently selected from H, CF₃, C₁₋₄alkyl, C₁₋₄alkoxy, F, Cl, Br, I, OH, NO₂ or NH₂; or, when R₁ and A are H, R₂ is (L)ₐ-(CH₂)_{b}-(T)_{c}-B wherein a, b, c, L, T and B are as defined above; and
R₁₇ is H, phenyl, benzyl or C₁₋₄alkyl;
which comprises:
a) reacting a compound of the formula: 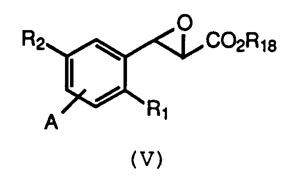 wherein:
A, R₁ and R₂ are as defined above; and
R₁₈ is C₁₋₄alkyl, phenyl, naphthyl, substituted phenyl, or substituted naphthyl, where the substituents may be one or two halogen, C₁₋₄alkyl, C₁₋₄alkoxy or trifluoromethyl groups; with a compound of the formula:
R₁₇NH₂
R₁₇ is H, phenyl, benzyl or C₁₋₄alkyl; or
b) reacting a compound of the formula: 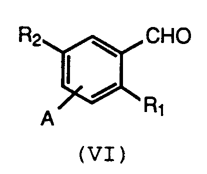 wherein A, R₁ and R₂ are as defined above,
with a compound of the formula:
X′-CH₂CONHR₁₇
wherein R₁₇ is as defined above and X′ is chloro, bromo or iodo.

10. A process for preparing a compound of the formula: 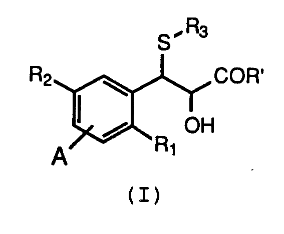 wherein:
R₁ is (L)ₐ-(CH₂)_{b}-(T)_{c}-B;
a is 0 or 1;
b is 3 to 14;
c is 0 or 1;
L and T are independently sulfur, oxygen, CH=CH, C≡C, or CH₂;
B is H, C₁₋₄alkyl, ethynyl, trifluoromethyl, isopropenyl, furanyl, thienyl, cyclohexyl or phenyl optionally monosubstituted with Br, Cl, CF₃, C₁₋₄alkoxy, C₁₋₄alkyl, methylthio or trifluoromethylthio;
R' is OH, NH₂, aryloxy or C₁₋₆alkoxy;
R₂ and A are independently selected from H, CF₃, C₁₋₄alkyl, C₁₋₄alkoxy, F, Cl, Br, I, OH, NO₂ or NH₂;or, when R₁ and A are H, R₂ is (L)ₐ-(CH₂)_{b}-(T)_{c}-B wherein a, b, c, L, T and B are as defined above;
R³ is (CH₂)ₙCH(R₅)COR₆, CH(CO₂H)CH₂CO₂H, CH₂CH₂Z, 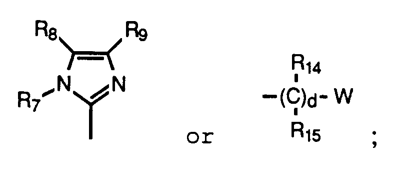 n is 0 to 6;
R₅ is hydrogen, amino, or NHCOCH₂CH₂CH(NH₂)CO₂H;
R₆ is hydroxy, amino, NHCH₂CO₂H or C₁₋₆alkoxy;
Z is SO₃H, SO₂NH₂ or CN;
R₇ is hydrogen, C₁₋₄alkyl or C₃₋₄alkenyl;
R₈ is hydrogen, C₁₋₄alkyl, carboxyl, carboxamido, or (CH₂)ₚCO₂R₁₂, wherein p is 1 or 2 and R₁₂ is C₁₋₆alkyl, or hydrogen when R₇ and R₉ are hydrogen or C₁₋₄alkyl;
R₉ is hydrogen, C₁₋₄alkyl, or (CH₂)ₚCO₂R₁₃, wherein p is 1 or 2 and R₁₃ is C₁₋₆alkyl or hydrogen, with the proviso that when n is 0, R₅ is hydrogen and further that R₇, R₈ and R₉ are not all hydrogen;
R₁₄ and R₁₅ are independently hydrogen or C₁₋₄alkyl at any point when d is not 0;
d is 0 to 6;
W is a six membered aryl or heteroaryl ring selected from phenyl, pyridyl, or pyrimidyl, unsubstituted or substituted with F, E, or D; or W is one of 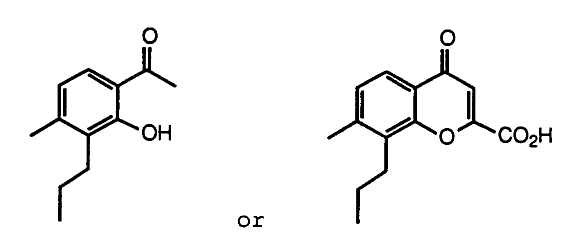 F is 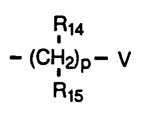 wherein R₁₄ and R₁₅ are independently hydrogen or C₁₋₄alkyl;
p is 0 to 6;
V is H, C₁₋₄alkyl, COR′, SO₃H, SO₂H, SO₂NH₂, COCH₂OH, CHOHCH₂OH, or tetrazolyl, with R′ as defined above; and
E and D are independently selected from H, OH, F, Cl, Br, CF₃, C₁₋₄alkyl, C₁₋₄alkoxy, methylthio, trifluoromethylthio, NO₂, NH₂, NHC₁₋₄alkyl, or C₁₋₄alkylCO-; which comprises reacting a compound of the formula: 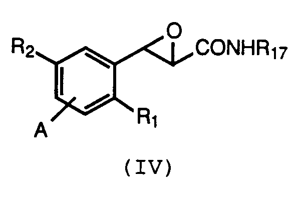 wherein:
R₁ is (L)ₐ-(CH₂)_{b}-(T)_{c}-B;
a is 0 or 1;
b is 3 to 14;
c is 0 or 1;
L and T are independently sulfur, oxygen, CH=CH, C≡C, or CH₂;
B is H, C₁₋₄alkyl, ethynyl, trifluoromethyl, isopropenyl, furanyl, thienyl, cyclohexyl or phenyl optionally monosubstituted with Br, Cl, CF₃, C₁₋₄alkoxy, C₁₋₄alkyl, methylthio or trifluoromethylthio;
R₂ and A are independently selected from H, CF₃, C₁₋₄alkyl, C₁₋₄alkoxy, F, Cl, Br, I, OH, NO₂ or NH₂; or, when R₁ and A are H, R₂ is (L)ₐ-(CH₂)_{b}-(T)_{c}-B wherein a, b, c, L, T and B are as defined above; and
R₁₇ is H, phenyl, benzyl or C₁₋₄alkyl;
with titanium isopropoxide and a compound of the formula:
R₃-S-M
wherein R₃ is as defined above and M is lithium, sodium or potassium; and
hydrolyzing the product thereof.

## Claims (Claims for the following Contracting State(s): ES)

1. A process for preparing a compound of the formula: 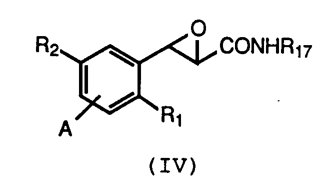 wherein:
R₁ is (L)ₐ-(CH₂)_{b}-(T)_{c}-B;
a is 0 or 1;
b is 3 to 14;
c is 0 or 1;
L and T are independently sulfur, oxygen, CH=CH, C≡C, or CH₂;
B is H, C₁₋₄alkyl, ethynyl, trifluoromethyl, isopropenyl, furanyl, thienyl, cyclohexyl or phenyl optionally monosubstituted with Br, Cl, CF₃, C₁₋₄alkoxy, C₁₋₄alkyl, methylthio or trifluoromethylthio;
R₂ and A are independently selected from H, CF₃, C₁₋₄alkyl, C₁₋₄alkoxy, F, Cl, Br, I, OH, NO₂ or NH₂; or, when R₁ and A are H, R₂ is (L)ₐ-(CH₂)_{b}-(T)_{c}-B wherein a, b, c, L, T and B are as defined above; and
R₁₇ is H, phenyl, benzyl or C₁₋₄alkyl;
which comprises:
a) reacting a compound of the formula: 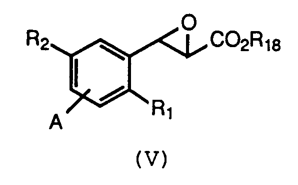 wherein:
A, R₁ and R₂ are as defined above; and
R₁₈ is C₁₋₄alkyl, phenyl, naphthyl, substituted phenyl, or substituted naphthyl, where the substituents may be one or two halogen, C₁₋₄alkyl, C₁₋₄alkoxy or trifluoromethyl groups; with a compound of the formula:
R₁₇NH₂
R₁₇ is H, phenyl, benzyl or C₁₋₄alkyl; or
b) reacting a compound of the formula: 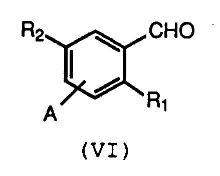 wherein A, R₁ and R₂ are as defined above,
with a compound of the formula:
X′-CH₂CONHR₁₇
wherein R₁₇ is as defined above and X′ is chloro, bromo or iodo.

2. A process according to claim 1 in which R₁ is C₈₋₁₃alkyl, C₇₋₁₂alkoxy, C₇₋₁₂alkylthio, C₁₀₋₁₂1-alkynyl, phenylC₄₋₁₀alkyl, phenylC₃₋₉alkoxy, phenylthioC₃₋₉alkyl, 10-undecynyloxy or 11-dodecynyloxy.

3. A process according to claim 1 in which R₂ is bromo, chloro, methyl, trifluoromethyl, hydroxy, methoxy, or nitro, or when R₁ and A are hydrogen, R₂ is C₈₋₁₃ alkyl, C₇₋₁₂alkoxy, C₇₋₁₂alkylthio, C₁₀₋₁₂1-alkynyl, phenylC₄₋₁₀alkyl, phenylC₃₋₉alkoxy, phenylthioC₃₋₉alkyl, 10-undecynyloxy or 11-dodecynyloxy.

4. A process according to claim 1 in which A and R₂ are both H.

5. A process according to claim 1 in which R₁₇ is H.

6. A compound according to claim 2 in which R₁ is C₁₁₋₁₃alkyl, C₁₀₋₁₂alkoxy or phenylC₇₋₉alkyl

7. A compound according to claim 6 in which R₁ is phenyloctyl.

8. A compound according to claim 7 which is (2R-trans)-3-[2-(8-phenyloctyl)phenyl]oxiranecarboxamide.

9. A process for preparing a compound of the formula: 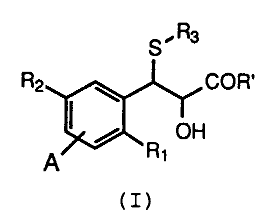 wherein:
R₁ is (L)ₐ-(CH₂)_{b}-(T)_{c}-B;
a is 0 or 1;
b is 3 to 14;
c is 0 or 1;
L and T are independently sulfur, oxygen, CH=CH, C≡C, or CH₂;
B is H, C₁₋₄alkyl, ethynyl, trifluoromethyl, isopropenyl, furanyl, thienyl, cyclohexyl or phenyl optionally monosubstituted with Br, Cl, CF₃, C₁₋₄alkoxy, C₁₋₄alkyl, methylthio or trifluoromethylthio;
R′ is OH, NH₂, aryloxy or C₁₋₆alkoxy;
R₂ and A are independently selected from H, CF₃, C₁₋₄alkyl, C₁₋₄alkoxy, F, Cl, Br, I, OH, NO₂ or NH₂; or, when R₁ and A are H, R₂ is (L)ₐ-(CH₂)_{b}-(T)_{c}-B wherein a, b, c, L, T and B are as defined above;
R³ is (CH₂)ₙCH(R₅)COR₆, CH(CO₂H)CH₂CO₂H, CH₂CH₂Z, 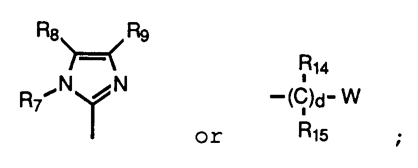 n is 0 to 6;
R₅ is hydrogen, amino, or NHCOCH₂CH₂CH(NH₂)CO₂H;
R₆ is hydroxy, amino, NHCH₂CO₂H or C₁₋₆alkoxy;
Z is SO₃H, SO₂NH₂ or CN;
R₇ is hydrogen, C₁₋₄alkyl or C₃₋₄alkenyl;
R₈ is hydrogen, C₁₋₄alkyl, carboxyl, carboxamido, or (CH₂)ₚCO₂R₁₂, wherein p is 1 or 2 and R₁₂ is C₁₋₆alkyl or hydrogen when R₇ and R₉ are hydrogen or C₁₋₄alkyl;
R₉ is hydrogen, C₁₋₄alkyl, or (CH₂)ₚCO₂R₁₃, wherein p is 1 or 2 and R₁₃ is C₁₋₆alkyl or hydrogen, with the proviso that when n is 0, R₅ is hydrogen and further that R₇, R₈ and R₉ are not all hydrogen;
R₁₄ and R₁₅ are independently hydrogen or C₁₋₄alkyl at any point when d is not 0;
d is 0 to 6;
W is a six membered aryl or heteroaryl ring selected from phenyl, pyridyl, or pyrimidyl, unsubstituted or substituted with F, E, or D; or W is one of 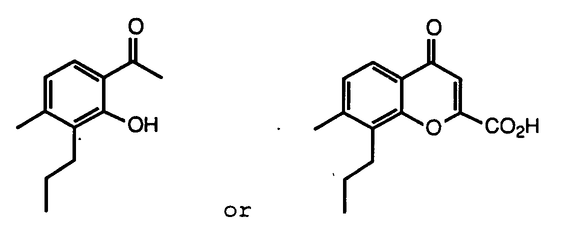 F is 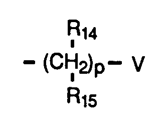 wherein R₁₄ and R₁₅ are independently hydrogen or C₁₋₄alkyl;
p is 0 to 6;
V is H, C₁₋₄alkyl, COR′, SO₃H, SO₂H, SO₂NH₂, COCH₂OH, CHOHCH₂OH, or tetrazolyl, with R′ as defined above; and
E and D are independently selected from H, OH, F, Cl, Br, CF₃, C₁₋₄alkyl, C₁₋₄alkoxy, methylthio, trifluoromethylthio, NO₂, NH₂, NHC₁₋₄alkyl, or C₁₋₄alkylCO-; which comprises reacting a compound of the formula: 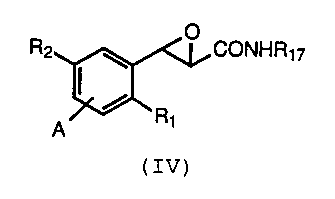 wherein:
R₁ is (L)ₐ-(CH₂)_{b}-(T)_{c}-B;
a is 0 or 1;
b is 3 to 14;
c is 0 or 1;
L and T are independently sulfur, oxygen, CH=CH, C≡C, or CH₂;
B is H, C₁₋₄alkyl, ethynyl, trifluoromethyl, isopropenyl, furanyl, thienyl, cyclohexyl or phenyl optionally monosubstituted with Br, Cl, CF₃, C₁₋₄alkoxy, C₁₋₄alkyl, methylthio or trifluoromethylthio;
R₂ and A are independently selected from H, CF₃, C₁₋₄alkyl, C₁₋₄alkoxy, F, Cl, Br, I, OH, NO₂ or NH₂; or, when R₁ and A are H, R₂ is (L)ₐ-(CH₂)_{b}-(T)_{c}-B wherein a, b, c, L, T and B are as defined above; and
R₁₇ is H, phenyl, benzyl or C₁₋₄alkyl;
with titanium isopropoxide and a compound of the formula:
R₃-S-M
wherein R₃ is as defined above and M is lithium, sodium or potassium; and
hydrolyzing the product thereof.

10. A process according to claim 9 for preparing (R*,S*) 2-hydroxy-3-[2-(carboxy)-ethylthio]-3-[2-(8-phenyloctyl) phenyl]-propanoic acid.

## Patentansprüche (Patentansprüche für folgende(n) Vertragsstaat(en): AT, BE, CH, DE, DK, FR, GB, GR, IT, LI, LU, NL, SE)

1. Verbindung der Formel 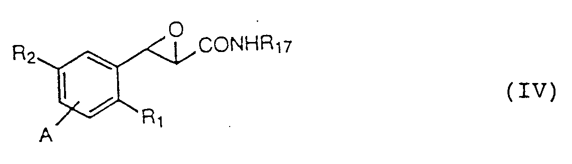 in der
R₁ (L)ₐ-(CH₂)_{b}-(T)_{c}-B ist;
a 0 oder 1 ist;
b 3 bis 14 ist;
c 0 oder 1 ist;
L und T unabhängig voneinander ein Schwefel- oder ein Sauerstoffatom, CH=CH, C≡C oder CH₂ bedeuten;
B ein Wasserstoffatom, einen C₁₋₄-Alkylrest, eine Ethinyl-, Trifluormethyl-, Isopropenyl-, Furanyl-, Thienyl-, Cyclohexyl- oder Phenylgruppe bedeutet, gegebenenfalls monosubstituiert mit Br, Cl, CF₃, C₁₋₄-Alkoxy, C₁₋₄-Alkyl, Methylthio oder Trifluormethylthio;
R₂ und A unabhängig voneinander ausgewählt sind aus einem Wasserstoffatom, CF₃, einem C₁₋₄-Alkyl- oder C₁₋₄-Alkoxyrest, einem Fluor-, Chlor-, Brom- oder Iodatom, OH, NO₂ oder NH₂; oder wenn R₁ und A ein Wasserstoffatom bedeuten, R₂ (L)ₐ-(CH₂)_{b}-(T)_{c}-B ist, wobei a, b, c, L, T und B wie vorstehend definiert sind; und
R₁₇ ein Wasserstoffatom, eine Phenyl- oder Benzylgruppe oder einen C₁₋₄-Alkylrest bedeutet.

2. Verbindung nach Anspruch 1, wobei R₁ einen C₈₋₁₃-Alkyl-, C₇₋₁₂-Alkoxy-, C₇₋₁₂-Alkylthio-, C₁₀₋₁₂-1-Alkinyl-, Phenyl-C₄₋₁₀-alkyl-, Phenyl-C₃₋₉-alkoxy-, Phenylthio-C₃₋₉-alkylrest, eine 10-Undecinyloxy- oder 11-Dodecinyloxygruppe bedeutet.

3. Verbindung nach Anspruch 1, wobei R₂ ein Brom- oder Chloratom, eine Methyl-, Trifluormethyl-, Hydroxy-, Methoxy- oder Nitrogruppe bedeutet, oder wenn R₁ und A Wasserstoffatome bedeuten, R₂ einen C₈₋₁₃-Alkyl-, C₇₋₁₂-Alkoxy- C₇₋₁₂-Alkylthio-, C₁₀₋₁₂-1-Alkinyl-, Phenyl-C₄#SB-#₁₀-alkyl-, Phenyl-C₃₋₉-alkoxy-, Phenylthio-C₃₋₉-alkylrest, eine 10-Undecinyloxy- oder 11-Dodecinyloxygruppe bedeutet.

4. Verbindung nach Anspruch 1, wobei A und R₂ beide Wasserstoffatome bedeuten.

5. Verbindung nach Anspruch 1, wobei R₁₇ ein Wasserstoffatom bedeutet.

6. Verbindung nach Anspruch 2, wobei R₁ einen C₁₁₋₁₃-Alkyl-, C₁₀₋₁₂-Alkoxy- oder Phenyl-C₇₋₉-alkylrest bedeutet.

7. Verbindung nach Anspruch 6, wobei R₁ eine Phenyloctylgruppe bedeutet.

8. Verbindung nach Anspruch 7, nämlich (2R-trans)-3-[2-(8-Phenyloctyl)phenyl]oxirancarboxamid.

9. Verfahren zur Herstellung einer Verbindung der Formel 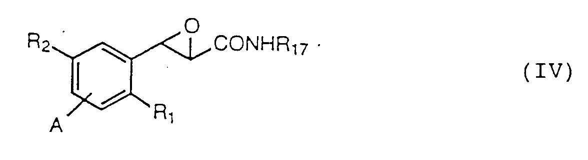 in der
R₁ (L)ₐ-(CH₂)_{b}-(T)_{c}-B ist;
a 0 oder 1 ist;
b 3 bis 14 ist;
c 0 oder 1 ist;
L und T unabhängig voneinander ein Schwefel- oder Sauerstoffatom, CH=CH, C≡C oder CH₂ bedeuten;
B ein Wasserstoffatom, einen C₁₋₄-Alkylrest, eine Ethinyl-, Trifluormethyl-, Isopropenyl-, Furanyl-, Thienyl-, Cyclohexyl- oder Phenylgruppe bedeutet, gegebenenfalls monosubstituiert mit Br, Cl, CF₃, C₁₋₄-Alkoxy, C₁₋₄-Alkyl, Methylthio oder Trifluormethylthio;
R₂ und A unabhängig voneinander ausgewählt sind aus einem Wasserstoffatom, CF₃, einem C₁₋₄-Alkyl- oder C₁₋₄-Alkoxyrest, einem Fluor-, Chlor-, Brom- oder Iodatom, OH, NO₂ oder NH₂; oder wenn R₁ und A Wasserstoffatome sind, R₂ (L)ₐ-(CH₂)_{b}-(T)_{c}-B ist, wobei a, b, c, L, T und B wie vorstehend definiert sind und
R₁₇ ein Wasserstoffatom, eine Phenyl- oder Benzylgruppe oder einen C₁₋₄-Alkylrest bedeutet; umfassend
a) Umsetzen einer Verbindung der Formel 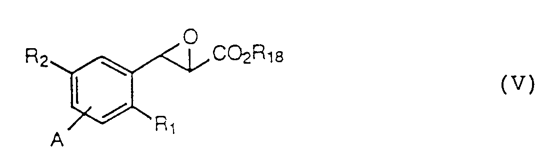 in der
A, R₁ und R₂ wie vorstehend definiert sind, und
R₁₈ einen C₁₋₄-Alkylrest, eine Phenyl-, Naphthyl-, substituierte Phenyl- oder substituierte Naphthylgruppe bedeutet, wobei die Substituenten ein oder zwei Halogenatome, C₁₋₄-Alkylreste, C₁₋₄-Alkoxyreste oder Trifluormethylgruppen bedeuten können, mit einer Verbindung der Formel
R₁₇NH₂
wobei R₁₇ ein Wasserstoffatom, eine Phenyl- oder Benzylgruppe oder einen C₁₋₄-Alkylrest bedeutet, oder
b) Umsetzen einer Verbindung der Formel 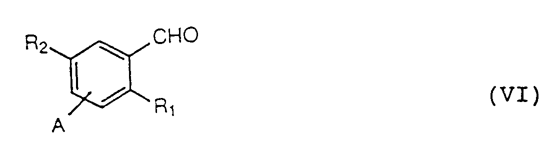 in der A, R₁ und R₂ wie vorstehend definiert sind, mit einer Verbindung der Formel
X'-CH₂CONHR₁₇
wobei R₁₇ wie vorstehend definiert ist und X' ein Chlor-, Brom- oder Iodatom bedeutet.

10. Verfahren zur Herstellung einer Verbindung der Formel 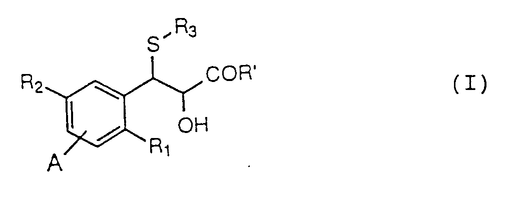 in der
R₁ (L)ₐ-(CH₂)_{b}-(T)_{c}-B ist;
a 0 oder 1 ist;
b 3 bis 14 ist;
c 0 oder 1 ist;
L und T unabhängig voneinander ein Schwefel- oder Sauerstoffatom, CH=CH, C≡C oder CH₂ bedeuten;
B ein Wasserstoffatom, einen C₁₋₄-Alkylrest oder eine Ethinyl-, Trifluormethyl-, Isopropenyl-, Furanyl-, Thienyl-, Cyclohexyl- oder Phenylgruppe bedeutet, gegebenenfalls monosubstituiert mit Br, Cl, CF₃, C₁₋₄-Alkoxy, C₁₋₄-Alkyl, Methylthio oder Trifluormethylthio;
R' OH, NH₂, einen Aryloxy- oder C₁₋₆-Alkoxyrest bedeutet,
R₂ und A unabhängig voneinander ausgewählt sind aus einem Wasserstoffatom, CF₃, einem C₁₋₄-Alkyl- oder C₁₋₄-Alkoxyrest, einem Fluor-, Chlor-, Brom- oder Iodatom, OH, NO₂ oder NH₂; oder wenn R₁ und A Wasserstoffatome sind, R₂ (L)ₐ-(CH₂)_{b}-(T)_{c}-B bedeutet, wobei a, b, c, L, T und B wie vorstehend definiert sind;
R₃ (CH₂)ₙCH(R₅)COR₆, CH(CO₂H)CH₂CO₂H, CH₂CH₂Z 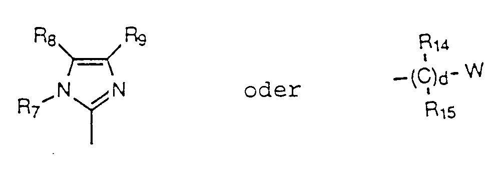 bedeutet,
n 0 bis 6 darstellt,
R₅ ein Wasserstoffatom, eine Aminogruppe oder NHCOCH₂CH₂CH(NH₂)CO₂H bedeutet;
R₆ eine Hydroxy- oder Aminogruppe, NHCH₂CO₂H oder einen C₁₋₆-Alkoxyrest bedeutet;
Z SO₃H, SO₂NH₂ oder CN bedeutet;
R₇ ein Wasserstoffatom, einen C₁₋₄-Alkyl- oder C₃₋₄-Alkenylrest bedeutet;
R₈ ein Wasserstoffatom, einen C₁₋₄-Alkylrest, eine Carboxylgruppe oder Carboxamidogruppe, oder (CH₂)ₚCO₂R₁₂ bedeutet, wobei p 1 oder 2 ist und R₁₂ einen C₁₋₆-Alkylrest oder ein Wasserstoffatom bedeutet, wenn R₇ und R₉ Wasserstoffatome oder C₁₋₄-Alkylrest bedeuten;
R₉ ein Wasserstoffatom, einen C₁₋₄-Alkyl-, oder (CH₂)ₚCO₂R₁₃-Rest bedeutet, wobei p 1 oder 2 ist und R₁₃ einen C₁₋₆-Alkylrest oder ein Wasserstoffatom bedeutet, mit der Maßgabe, daß, wenn n 0 ist, R₅ ein Wasserstoffatom bedeutet und zusätzlich, daß R₇, R₈ und R₉ nicht gleichzeitig Wasserstoffatome darstellen;
R₁₄ und R₁₅ unabhängig voneinander Wasserstoffatome oder C₁₋₄-Alkylreste an einem beliebigen Punkt darstellen, wenn d nicht 0 ist;
d 0 bis 6 ist;
W einen 6-gliedrigen Aryl- oder Heteroarylring bedeutet, ausgewählt aus Phenyl-, Pyridyl- oder Pyrimidylgruppen, unsubstituiert oder substituiert mit F, E oder D; oder W eine der Gruppen 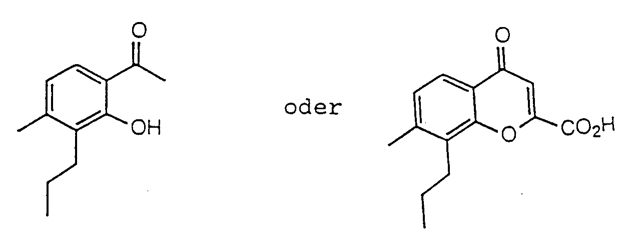 ist
F 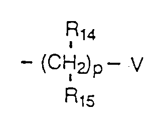 bedeutet,
wobei R₁₄ und R₁₅ unabhängig voneinander Wasserstoffatome oder C₁₋₄-Alkylreste darstellen;
p 0 bis 6 ist;
V ein Wasserstoffatom, einen C₁₋₄-Alkylrest, COR', SO₃H, SO₂H, SO₂NH₂, COCH₂OH, CHOHCH₂OH oder eine Tetrazolylgruppe bedeutet, wobei R' die vorstehend ausgewiesene Bedeutung aufweist, und
E und D unabhängig voneinander ausgewählt sind aus Wasserstoffatomen, OH, Fluor-, Chlor- oder Bromatomen, CF₃, C₁₋₄-Alkylresten, C₁₋₄-Alkoxyresten, Methylthiogruppen, Trifluormethylthiogruppen, NO₂, NH₂, NHC₁₋₄-Alkyl- oder C₁₋₄-AlkylCO-Resten, umfassend die Umsetzung einer Verbindung der Formel 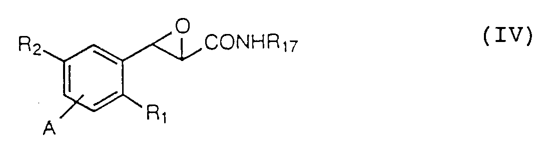 in der
R₁ (L)ₐ-(CH₂)_{b}-(T)_{c}-B ist;
a 0 oder 1 ist;
b 3 bis 14 ist;
c 0 oder 1 ist;
L und T unabhängig voneinander Schwefel- oder Sauerstoffatome, CH=CH, C≡C oder CH₂ bedeuten;
B ein Wasserstoffatom, einen C₁₋₄-Alkylrest, eine Ethinyl-, Trifluormethyl-, Isopropenyl-, Furanyl-, Thienyl-, Cyclohexyl- oder Phenylgruppe bedeutet, gegebenenfalls monosubstituiert mit Br, Cl, CF₃, C₁₋₄-Alkoxy, C₁₋₄-Alkyl, Methylthio oder Trifluormethylthio;
R₂ und A unabhängig voneinander ausgewählt sind aus einem Wasserstoffatom, CF₃, einem C₁₋₄-Alkyl- oder C₁₋₄-Alkoxyrest, einem Fluor-, Chlor-, Brom- oder Iodatom, OH, NO₂ oder NH₂; oder wenn R₁ und A Wasserstoffatome sind, R₂ (L)ₐ-(CH₂)_{b}-(T)_{c}-B bedeutet, wobei a, b, c, L, T und B wie vorstehend definiert sind; und
R₁₇ ein Wasserstoffatom, eine Phenyl- oder Benzylgruppe oder einen C₁₋₄-Alkylrest bedeutet;
mit Tritanisopropoxid und einer Verbindung der Formel
R₃-S-M
wobei R₃ wie vorstehend definiert ist und M Lithium, Natrium oder Kalium bedeutet; und
Hydrolysieren des Produktes davon.

## Patentansprüche (Patentansprüche für folgende(n) Vertragsstaat(en): ES)

1. Verfahren zur Herstellung einer Verbindung der Formel 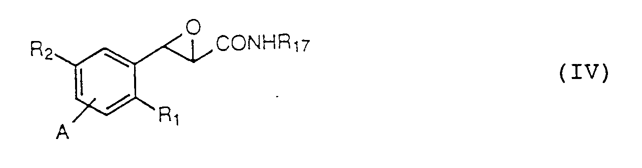 in der
R₁ (L)ₐ-(CH₂)_{b}-(T)_{c}-B ist;
a 0 oder 1 ist;
b 3 bis 14 ist;
c 0 oder 1 ist;
L und T unabhängig voneinander ein Schwefel- oder Sauerstoffatom, CH=CH, C≡C oder CH₂ bedeuten;
B ein Wasserstoffatom, einen C₁₋₄-Alkylrest, eine Ethinyl-, Trifluormethyl-, Isopropenyl-, Furanyl-, Thienyl-, Cyclohexyl- oder Phenylgruppe bedeutet, gegebenenfalls monosubstituiert mit Br, Cl, CF₃, C₁₋₄-Alkoxy, C₁₋₄-Alkyl, Methylthio oder Trifluormethylthio;
R₂ und A unabhängig voneinander ausgewählt sind aus einem Wasserstoffatom, CF₃, einem C₁₋₄-Alkyl- oder C₁₋₄-Alkoxyrest, einem Fluor-, Chlor-, Brom- oder Iodatom, OH, NO₂ oder NH₂; oder wenn R₁ und A Wasserstoffatome sind, R₂ (L)ₐ-(CH₂)_{b}-(T)_{c}-B ist, wobei a, b, c, L, T und B wie vorstehend definiert sind und
R₁₇ ein Wasserstoffatom, eine Phenyl- oder Benzylgruppe oder einen C₁₋₄-Alkylrest bedeutet; umfassend
a) Umsetzen einer Verbindung der Formel 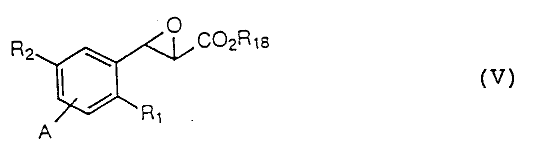 in der
A, R₁ und R₂ wie vorstehend definiert sind, und R₁₈ einen C₁₋₄-Alkylrest, eine Phenyl-, Naphthyl-, substituierte Phenyl- oder substituierte Naphthylgruppe bedeutet, wobei die Substituenten ein oder zwei Halogenatome, C₁₋₄-Alkylreste, C₁₋₄-Alkoxyreste oder Trifluormethylgruppen bedeuten können, mit einer Verbindung der Formel
R₁₇NH₂
wobei R₁₇ ein Wasserstoffatom, eine Phenyl- oder Benzylgruppe oder einen C₁₋₄-Alkylrest bedeutet, oder
b) Umsetzen einer Verbindung der Formel 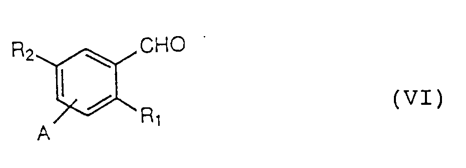 in der A, R₁ und R₂ wie vorstehend definiert sind, mit einer Verbindung der Formel
X'-CH₂CONHR₁₇
wobei R₁₇ wie vorstehend definiert ist und X' ein Chlor-, Brom- oder Iodatom bedeutet.

2. Verfahren nach Anspruch 1, wobei R₁ einen C₈-₁₃-Alkyl-, C₇₋₁₂-Alkoxy-, C₇₋₁₂-Alkylthio-, C₁₀₋₁₂-1-Alkinyl-, Phenyl-C₄₋₁₀-alkyl-, Phenyl-C₃₋₉-alkoxy-, Phenylthio-C₃₋₉-alkylrest, eine 10-Undecinyloxy- oder 11-Dodecinyloxygruppe bedeutet.

3. Verfahren nach Anspruch 1, wobei R₂ ein Brom- oder Chloratom, eine Methyl-, Trifluormethyl-, Hydroxy-, Methoxy- oder Nitrogruppe bedeutet, oder wenn R₁ und A Wasserstoffatome bedeuten, R₂ einen C₈₋₁₃-Alkyl-, C₇₋₁₂-Alkoxy-, C₇₋₁₂-Alkylthio-, C₁₀₋₁₂-1-Alkinyl-, Phenyl-C₄₋₁₀-alkyl-, Phenyl-C₃₋₉-alkoxy-, Phenylthio-C₃₋₉-alkylrest, eine 10-Undecinyloxy- oder 11-Dodecinyloxygruppe bedeutet.

4. Verfahren nach Anspruch 1, wobei A und R₂ beide Wasserstoffatome bedeuten.

5. Verfahren nach Anspruch 1, wobei R₁₇ ein Wasserstoffatom bedeutet.

6. Verfahren nach Anspruch 2, wobei R₁ einen C₁₁₋₁₃-Alkyl-, C₁₀₋₁₂-Alkoxy- oder Phenyl-C₇₋₉-alkylrest bedeutet.

7. Verfahren nach Anspruch 6, wobei R₁ eine Phenyloctylgruppe bedeutet.

8. Verfahren nach Anspruch 7, nämlich (2R-trans)-3-[2-(8-Phenyloctyl)phenyl]oxirancarboxamid.

9. Verfahren zur Herstellung einer Verbindung der Formel 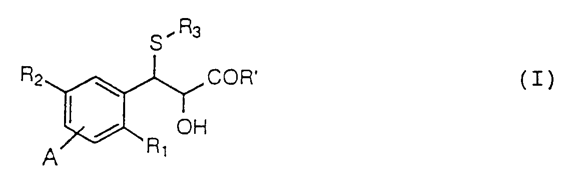 in der
R₁ (L)ₐ-(CH₂)_{b}-(T)_{c}-B ist;
a 0 oder 1 ist;
b 3 bis 14 ist;
c 0 oder 1 ist;
L und T unabhängig voneinander ein Schwefel- oder Sauerstoffatom, CH=CH, C≡C oder CH₂ bedeuten;
B ein Wasserstoffatom, einen C₁₋₄-Alkylrest oder eine Ethinyl-, Trifluormethyl-, Isopropenyl-, Furanyl-, Thienyl-, Cyclohexyl- oder Phenylgruppe bedeutet, gegebenenfalls monosubstituiert mit Br, Cl, CF₃, C₁₋₄-Alkoxy, C₁₋₄-Alkyl, Methylthio oder Trifluormethylthio;
R' OH, NH₂, einen Aryloxy- oder C₁₋₆-Alkoxyrest bedeutet,
R₂ und A unabhängig voneinander ausgewählt sind aus einem Wasserstoffatom, CF₃, einem C₁₋₄-Alkyl- oder C₁₋₄-Alkoxyrest, einem Fluor-, Chlor-, Brom- oder Iodatom, OH, NO₂ oder NH₂; oder wenn R₁ und A Wasserstoffatome sind, R₂ (L)ₐ-(CH₂)_{b}-(T)_{c}-B bedeutet, wobei a, b, c, L, T und B wie vorstehend definiert sind;
R₃ (CH₂)ₙCH(R₅)COR₆, CH(CO₂H)CH₂CO₂H, CH₂CH₂Z 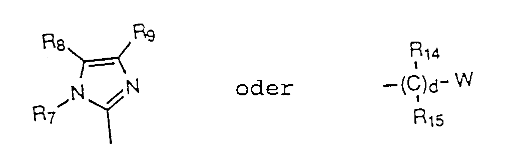 bedeutet,
n 0 bis 6 darstellt,
R₅ ein Wasserstoffatom, eine Aminogruppe oder NHCOCH₂CH₂CH(NH₂)CO₂H bedeutet;
R₆ eine Hydroxy- oder Aminogruppe, NHCH₂CO₂H oder einen C₁₋₆-Alkoxyrest bedeutet;
Z SO₃H, SO₂NH₂ oder CN bedeutet;
R₇ ein Wasserstoffatom, einen C₁₋₄-Alkyl- oder C₃₋₄-Alkenylrest bedeutet;
R₈ ein Wasserstoffatom, einen C₁₋₄-Alkylrest, eine Carboxylgruppe oder Carboxamidogruppe, oder (CH₂)ₚCO₂R₁₂ bedeutet, wobei p 1 oder 2 ist und R₁₂ einen C₁₋₆-Alkylrest oder ein Wasserstoffatom bedeutet, wenn R₇ und R₉ Wasserstoffatome oder C₁₋₄-Alkylrest bedeuten;
R₉ ein Wasserstoffatom, einen C₁₋₄-Alkyl-, oder (CH₂)ₚCO₂R₁₃-Rest bedeutet, wobei p 1 oder 2 ist und R₁₃ einen C₁₋₆-Alkylrest oder ein Wasserstoffatom bedeutet, mit der Maßgabe, daß, wenn n 0 ist, R₅ ein Wasserstoffatom bedeutet und zusätzlich, daß R₇, R₈ und R₉ nicht gleichzeitig Wasserstoffatome darstellen;
R₁₄ und R₁₅ unabhängig voneinander Wasserstoffatome oder C₁₋₄-Alkylreste an einem beliebigen Punkt darstellen, wenn d nicht 0 ist;
d 0 bis 6 ist;
W einen 6-gliedrigen Aryl- oder Heteroarylring bedeutet, ausgewählt aus Phenyl-, Pyridyl- oder Pyrimidylgruppen, unsubstituiert oder substituiert mit F, E oder D; oder W eine der Gruppen 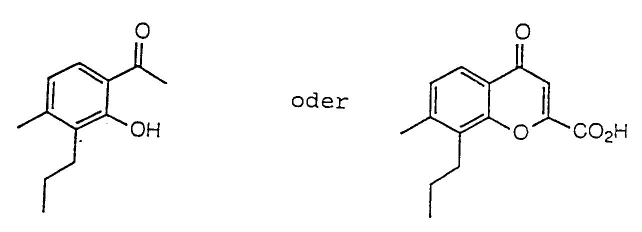 ist
F 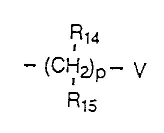 bedeutet,
wobei R₁₄ und R₁₅ unabhängig voneinander Wasserstoffatome oder C₁₋₄-Alkylreste darstellen;
p 0 bis 6 ist;
V ein Wasserstoffatom, einen C₁₋₄-Alkylrest, COR', SO₃H, SO₂H, SO₂NH₂, COCH₂OH, CHOHCH₂OH oder eine Tetrazolylgruppe bedeutet, wobei R' die vorstehend ausgewiesene Bedeutung aufweist, und
E und D unabhängig voneinander ausgewählt sind aus Wasserstoffatomen, OH, Fluor-, Chlor- oder Bromatomen, CF₃, C₁₋₄-Alkylresten, C₁₋₄-Alkoxyresten, Methylthiogruppen, Trifluormethylthiogruppen, NO₂, NH₂, NHC₁₋₄-Alkyl- oder C₁₋₄-AlkylCO-Resten, umfassend die Umsetzung einer Verbindung der Formel 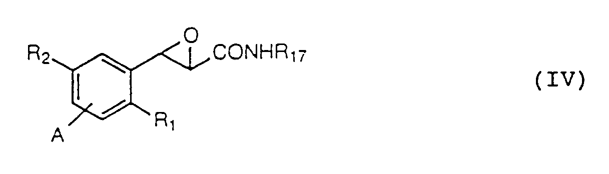 in der
R₁ (L)ₐ-(CH₂)_{b}-(T)_{c}-B ist;
a 0 oder 1 ist;
b 3 bis 14 ist;
c 0 oder 1 ist;
L und T unabhängig voneinander Schwefel- oder Sauerstoffatome, CH=CH, C≡C oder CH₂ bedeuten;
B ein Wasserstoffatom, einen C₁₋₄-Alkylrest, eine Ethinyl-, Trifluormethyl-, Isopropenyl-, Furanyl-, Thienyl-, Cyclohexyl- oder Phenylgruppe bedeutet, gegebenenfalls monosubstituiert mit Br, Cl, CF₃, C₁₋₄-Alkoxy, C₁₋₄-Alkyl, Methylthio oder Trifluormethylthio;
R₂ und A unabhängig voneinander ausgewählt sind aus einem Wasserstoffatom, CF₃, einem C₁₋₄-Alkyl- oder C₁₋₄-Alkoxyrest, einem Fluor-, Chlor-, Brom- oder Iodatom, OH, NO₂ oder NH₂; oder wenn R₁ und A Wasserstoffatome sind, R₂ (L)ₐ-(CH₂)_{b}-(T)_{c}-B bedeutet, wobei a, b, c, L, T und B wie vorstehend definiert sind; und
R₁₇ ein Wasserstoffatom, eine Phenyl- oder Benzylgruppe oder einen C₁₋₄-Alkylrest bedeutet;
mit Titanisopropoxid und einer Verbindung der Formel
R₃-S-M
wobei R₃ wie vorstehend definiert ist und M Lithium, Natrium oder Kalium bedeutet; und
Hydrolysieren des Produktes davon.

10. Verfahren nach Anspruch 9 zur Herstellung von (R*,S*)-2-Hydroxy-3-[2-(carboxy)-ethylthio]-3-[2(8-phenyloctyl)phenyl]-propansäure.

## Revendications (Revendications pour l'(les) Etat(s) contractant(s) suivant(s): AT, BE, CH, DE, DK, FR, GB, GR, IT, LI, LU, NL, SE)

1. Composé de formule : 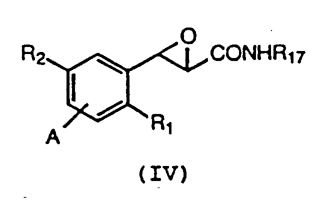 dans lequel :
- R₁ représente un groupe (L)ₐ-(CH₂)_{b}-(T)_{c}-B:
- a est égal à 0 ou à 1;
- b est compris entre 3 et 14;
- c est égal à 0 ou à 1;
- L et T sont, indépendamment, des atomes de soufre , d'oxygène des groupes CH=CH; C≡C, ou CH₂;
- B est un atome d'hydrogène un groupe alkyle C₁₋₄, ethinyle, trifluorométhyle, isopropényle, furanyle, thiényle, cyclohexyle, ou phényle, éventuellement monosubstitué par Br, Cl, CF₃, un groupe alcoxy C₁₋₄, alkyle C₁₋₄, méthylthio ou trifluorométhylthio;
- R₂ et A sont choisis indépendamment parmi les groupes H, CF₃, alkyle C₁₋₄, alcoxy C₁₋₄, F, Cl, Br, I, OH, NO₂, ou NH₂; ou, lorsque R₁ et A sont des atomes d'hydrogène; R₂ est un groupe (L)ₐ-(CH₂)_{b}-(T)_{c}-B dans lequel a,b c, L, T, et B sont tels que définis ci-dessus; et
- R₁₇ est un atome d'hydrogène, un groupe phényle, benzyle ou alkyle C₁₋₄.

2. Composé suivant la revendication 1, dans lequel R₁ est un groupe alkyle C₈₋₁₃, alcoxy C₇₋₁₂, alkylC₇₋₁₂thio, 1-alkynyle C₁₀₋ ₁₂, phénylalkyle C₄₋₁₀, phénylalcoxy C₃₋₉, phénylthioalkyle C₃₋₉, 10-undécynyloxy, ou 11-dodécynyloxy.

3. Composé suivant la revendication 1, dans lequel R₂ est bromo, chloro, méthyle, trifluorométhyle hydroxy, méthoxy, ou nitro, ou lorsque R₁ et A sont des atomes d'hydrogène, R₂ est un groupe alkyle C₈₋₁₃, alcoxy C₇₋₁₂, alkylC₇₋₁₂thio, 1-alkynl C₁₀₋₁₂, phénylalkyle C₄₋₁₀, phénylalcoxy C₃₋₉, phénylthioalkyle C₃₋₉, 10-undécynyloxy, ou 11-dodécynyloxy.

4. Composé suivant la revendication 1, dans lequel A et R₂ sont tous deux des atomes d'hydrogène.

5. Composé suivant la revendication 1, dans lequel R₁₇ est un atome d'hydrogène.

6. Composé suivant la revendication 2, dans lequel R₁ est un groupe alkyle C₁₁₋₁₃, alcoxy C₁₀₋₁₂, ou phénylalkyle C₇₋₉.

7. Composé suivant la revendication 6, dans lequel R₁ est un groupe phényloctyle.

8. Composé suivant la revendication 7, qui est le (2R-trans)-3-[2-(8-phényloctyl)phényl]oxiranecarboxamide.

9. Procédé de préparation d'un composé de formule : 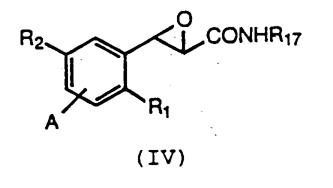 dans lequel :
- R₁ est un groupe (L)ₐ-(CH₂)_{b}-(T)_{c}-B;
- a est égal à 0 ou à 1;
- b est compris entre 3 et 14;
- c est égal à 0 ou à 1;
- L et T sont indépendamment, des atomes de soufre, d'oxygène, des groupes CH=CH; C≡C, ou CH₂;
- B est un atome d'hydrogène, un groupe alkyle C₁₋₄, ethinyle, trifluorométhyle, isopropényle, furanyle, thiényle, cyclohexyle, ou phényle, éventuellement monosubstitué par Br, Cl, CF₃, un groupe alcoxy C₁₋₄, alkyle C₁₋₄, méthylthio ou trifluorométhylthio;
- R₂ et A sont choisis indépendamment parmi les groupes H, CF₃, alkyle C₁₋₄, alcoxy C₁₋₄, F, Cl, Br, I, OH, NO₂, ou NH₂, ou, lorsque R₁ et A sont des atomes d'hydrogène, R₂ est un groupe (L)ₐ-(CH₂)_{b}-(T)_{c}-B dans lequel a,b c, L, T, et B sont tels que définis ci-dessus; et
- R₁₇ est un atome d'hydrogène, un groupe phényle benzyle ou alkyle C₁₋₄;
qui comprend :
(a) la réaction d'un composé de formule : 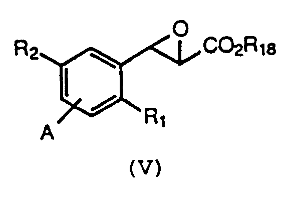 dans lequel :
- A, R₁, et R₂ sont tels que définis plus haut; et
- R₁₈ est un groupe alkyle C₁₋₄, phényle naphtyle, phényle substitué, ou naphtyle substitué, dans lesquels les substituants peuvent être un ou deux halogènes des groupes alkyle C₁₋₄, alcoxy C₁₋₄ ou trifluorométhyle; avec un composé de formule:
R₁₇NH₂
dans lequel R₁₇ est un atome d'hydrogène, un groupe phényle, benzyle ou alkyle C₁₋₄, ou
(b) la réaction d'un composé de formule: 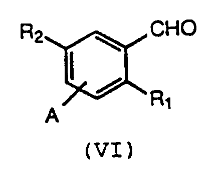 dans lequel A, R₁ et R₂ sont tels que définis plus haut: avec un composé de formule :
X'-CH₂CONHR₁₇
dans lequel R₁₇ est tel que défini plus haut et X' est un groupe chloro, bromo, ou iodo.

10. Procédé de préparation d'un composé de formule : 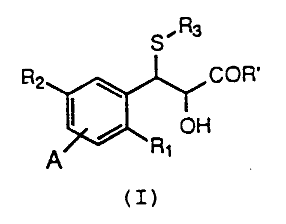 dans lequel :
-- R₁ représente un groupe (L)ₐ-(CH₂)_{b}-(T)_{c}-B;
- a est égal à 0 ou à 1;
- b est compris entre 3 et 14;
- c est égal à 0 ou à 1;
- L et T sont, indépendamment des atomes de soufre, d'oxygène, des groupes CH=CH; C≡C, ou CH₂;
- B est un atome d'hydrogène, un groupe alkyle C₁₋₄, ethinyle, trifluorométhyle, isopropényle, furanyle, thiényle, cyclohexyle, ou phényle, éventuellement monosubstitué par Br, Cl, CF₃, un groupe alcoxy C₁₋₄, alkyle C₁₋₄, méthylthio ou trifluorométhylthio;
- R' est un groupe hydroxyle, NH₂, aryloxy ou alcoxy C₁₋₆;
- R₂ et A sont choisis indépendamment parmi les groupes H, CF₃, alkyle C₁₋₄, alcoxy C₁₋₄, F, Cl, Br, I, OH, NO₂, ou NH₂; ou, lorsque R¹ et A sont des atomes d'hydrogène, R₂ est un groupe (L)ₐ-(CH₂)_{b}-(T)_{c}-B dans lequel a,b c, L, T et B sont tels que définis ci-dessus;
- R₃ est un groupe (CH₂)ₙCH(R₅)COR₆, CH(CO₂H)CH₂CO₂H, CH₂CH₂Z, 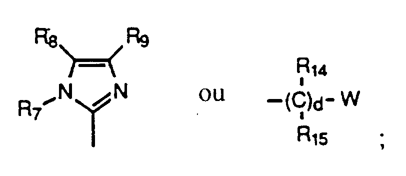
- n est compris entre 0 et 6;
- R₅ est un atome d'hydrogène un groupe amino, ou un groupe NHCOCH₂CH₂CH(NH₂)CO₂H;
- R₆ est un groupe hydroxy, amino, ou NHCH₂CO₂H ou alcoxy C₁₋₆;
- Z est un groupe SO₃H, SO₂NH₂, ou CN;
- R₇ est un atome d'hydrogène, un groupe alkyle C₁₋₄ ou alkényle C₃₋₄;
- R₈ est un atome d'hydrogène, un groupe alkyle C₁₋₄, carboxyle, carboxamido, ou (CH₂)ₚCO₂R₁₂, dans lequel p est égal à 1 ou à 2, et R₁₂ représente un groupe alkyle C₁₋₆ ou un atome d'hydrogène lorsque R₇ et R₉ sont des atomes d'hydrogène ou des groupes alkyle C₁₋₄;
- R₉ est un atome d'hydrogène, un groupe alkyle C₁₋₄, ou (CH₂)ₚCO₂R₁₃, dans lequel p est égal à 1 ou à 2, et R₁₃ est un groupe alkyle C₁₋₆, ou un atome d'hydrogène, à condition que lorsque n est égal à 0, R₅ est un atome d'hydrogène et que de plus, R₇, R₈ et R₉ ne sont pas tous des atomes d'hydrogène;
- R₁₄ et R₁₅ sont indépendamment des atomes d'hydrogène ou des groupes alkyle C₁₋₄, en chaque point lorsque d est n'est pas égal à 0;
- d est compris entre 0 et 6;
- W est un cycle à 6 atomes, aryle ou hétéroaryle, choisi parmi les groupes phényle, pyridyle, ou pyrimidyle, non substitué ou substitué par F, E, ou D; ou W est un des groupes 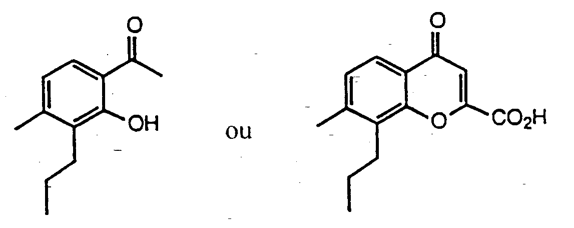
- F est un groupe : 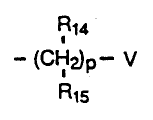 dans lequel R₁₄ et R₁₅ sont indépendamment des atomes d'hydrogène ou des groupes alkyle C₁₋₄,
- p est compris entre 0 et 6;
- V est un atome d'hydrogène, un groupe alkyle C₁₋₄, COR', SO₃H, SO₂H, SO₂NH₂, COCH₂OH, CHOHCH₂OH, ou tétrazolyle, avec R' tel que défini plus haut; et
- E et D sont indépendamment, choisis parmi les groupes H, OH, F, Cl, Br, CF₃, alkyle C₁₋₄, alcoxy C₁₋₄, méthylthio, trifluorométhylthio, NO₂, NH₂, NHalkyle C₁₋₄, ou alkylC₁₋₄CO-;
lequel procédé comprend la réaction d'un composé de formule : 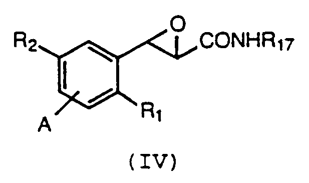 dans lequel :
- R₁ représente un groupe (L)ₐ-(CH₂)_{b}-(T)_{c}-B;
- a est égal à 0 ou à 1;
- b est compris entre 3 et 14;
- c est égal à 0 ou à 1;
- L et T sont, indépendamment, des atomes de soufre, d'oxygène, des groupes CH=CH; C≡C, ou CH₂;
- B est un atome d'hydrogène, un groupe alkyle C₁₋₄, ethinyle, trifluorométhyle, isopropényle, furanyle, thiényle, cyclohexyle, ou phényle, éventuellement monosubstitué par Br, Cl, CF₃, un groupe alcoxy C₁₋₄, alkyle C₁₋₄, méthylthio ou trifluorométhylthio;
- R₂ et A sont choisis indépendamment parmi les groupes H, CF₃, alkyle C₁₋₄, alcoxy C₁₋₄, F, Cl, Br, I, OH, NO₂, ou NH₂; ou, lorsque R₁ et A sont des atomes d'hydrogène, R₂ est un groupe (L)ₐ-(CH₂)_{b}-(T)_{c}-B dans lequel a,b c, L, T, et B sont tels que définis ci-dessus; et
- R₁₇ est un atome d'hydrogène, un groupe phényle, benzyle ou alkyle C₁₋₄;
avec l'isopropoxyde de titane et un composé de formule :
R₃-S-M
dans lequel R₃ est tel que défini plus haut, et M représente le lithium, le sodium ou le potassium; et
I'hydrolyse du produit obtenu.

## Revendications (Revendications pour l'(les) Etat(s) contractant(s) suivant(s): ES)

1. Procédé de préparation d'un composé de formule : 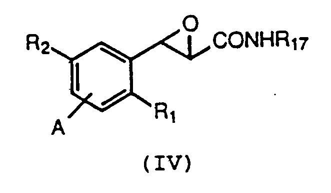 dans lequel :
- R₁ représente un groupe (L)ₐ-(CH₂)_{b}-(T)_{c}-B;
- a est égal à 0 ou à 1;
- b est compris entre 3 et 14;
- c est égal à 0 ou à 1;
- L et T sont, indépendamment, des atomes de soufre , d'oxygène, des groupes CH=CH; C≡C, ou CH₂;
- B est un atome d'hydrogène, un groupe alkyle C₁₋₄, ethinyle, trifluorométhyle, isopropényle, furanyle, thiényle cyclohexyle, ou phényle, éventuellement monosubstitué par Br, Cl, CF₃, un groupe alcoxy C₁₋₄, alkyle C₁₋₄, méthylthio ou trifluorométhylthio;
- R₂ et A sont choisis indépendamment parmi les groupes H, CF₃, alkyle C₁₋₄, alcoxy C₁₋₄, F, Cl, Br, I, OH, NO₂ ou NH₂; ou, lorsque R₁ et A sont des atomes d'hydrogène, R₂ est un groupe (L)ₐ-(CH₂)_{b}-(T)_{c}-B dans lequel a,b c, L, T, et B sont tels que définis ci-dessus; et
- R₁₇ est un atome d'hydrogène, un groupe phényle, benzyle ou alkyle C₁₋₄.
qui comprend :
(a) la réaction d'un composé de formule : 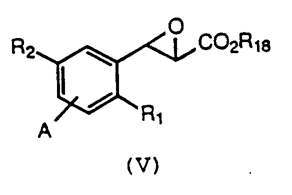 dans lequel :
- A, R₁, et R₂ sont tels que définis plus haut; et
- R₁₈ est un groupe alkyle C₁₋₄, phényle, naphtyle, phényle substitué, ou naphtyle substitué, dans lesquels les substituants peuvent être un ou deux halogènes, des groupes alkyle C₁₋₄, alcoxy C₁₋₄ ou trifluorométhyle; avec un composé de formule :
R₁₇NH₂
dans lequel R₁₇ est un atome d'hydrogène un groupe phényle, benzyle ou alkyle C₁₋₄, ou
(b) la réaction d'un composé de formule : 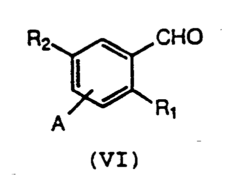 dans lequel A, R₁ et R₂ sont tels que définis plus haut; avec un composé de formule :
X'-CH₂CONHR₁₇
dans lequel R₁₇ est tel que défini plus haut et X' est un groupe chloro, bromo, ou iodo.

2. Procédé suivant la revendication 1, dans lequel R₁ est un groupe alkyle C₈₋₁₃, alcoxy C₇₋₁₂, alkylC₇₋₁₂thio, 1-alkynyle C₁₀₋ ₁₂, phénylalkyle C₄₋₁₀, phénylalcoxy C₃₋₉, phénylthioallkyle C₃₋₉, 10-undécynyloxy ou 11-dodécynyloxy.

3. Procédé suivant la revendication 1, dans lequel R₂ est bromo, chloro, méthyle, trifluorométhyle, hydroxy, méthoxy, ou nitro, ou lorsque R₁ et A sont des atomes d'hydrogène, R₂ est un groupe alkyle C₈₋₁₃, alcoxy C₇₋₁₂, alkylC₇₋₁₂thio, 1-alkynyle C₁₀₋₁₂, phénylalkyle C₄₋₁₀, phénylalcoxy C₃₋₉, phénylthioalkyle C₃₋₉, 10-undécynyloxy, ou 11-dodécynyloxy.

4. Procédé suivant la revendication 1, dans lequel A et R₂ sont tous deux des atomes d'hydrogène.

5. Procédé suivant la revendication 1, dans lequel R₁₇ est un atome d'hydrogène.

6. Composé suivant la revendication 2, dans lequel R₁ est un groupe alkyle C₁₁₋₁₃, alcoxy C₁₀₋₁₂, ou phénylalkyle C₇₋₉.

7. Composé suivant la revendication 6, dans lequel R₁ est un groupe phényloctyle.

8. Composé suivant la revendication 7, qui est le (2R-trans)-3-[2-(8-phényloctyl)phényl]oxiranecarboxamide.

9. Procédé de préparation d'un composé de formule : 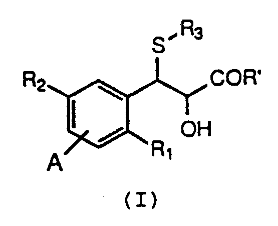 dans lequel :
- R₁ est un groupe (L)ₐ-(CH₂)_{b}-(T)_{c}-B;
- a est égal à 0 ou à 1:
- b est compris entre 3 et 14;
- c est égal à 0 ou à 1;
- L et T sont, indépendamment, des atomes de soufre , d'oxygène, des groupes CH=CH; C≡C, ou CH₂;
- B est un atome d'hydrogène, un groupe alkyle C₁₋₄ ethinyle, trifluorométhyle, isopropényle, furanyle, thiényle, cyclohexyle, ou phényle, éventuellement monosubstitué par Br, Cl, CF₃, un groupe alcoxy C₁₋₄, alkyle C₁₋₄, méthylthio ou trifluorométhylthio;
- R' est un groupe OH, NH₂, aryloxy ou alcoxy C₁₋₆;
- R₂ et A sont choisis indépendamment parmi les groupes H, CF₃, alkyle C₁₋₄, alcoxy C₁₋₄, F, Cl, Br, I, OH, NO₂, ou NH₂; ou lorsque R₁ et A sont des atomes d'hydrogène, R₂ est un groupe (L)ₐ-(CH₂)_{b}-(T)_{c}-B dans lequel a,b c, L, T, et B sont tels que définis ci-dessus;
- R₃ est un groupe (CH₂)ₙCH(R₅)COR₆, CH(CO₂H)CH₂CO₂H, CH₂CH₂Z, 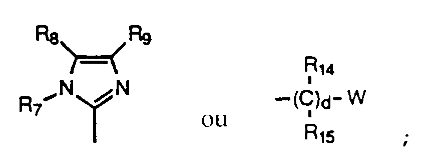
- n est compris entre 0 et 6;
- R₅ est un atome d'hydrogène, un groupe amino, ou un groupe NHCOCH₂CH₂CH(NH₂)CO₂H;
- R₆ est un groupe hydroxy amino, ou NHCH₂CO₂H ou alcoxy C₁₋₆;
- Z est un groupe SO₃H, SO₂NH₂, ou CN;
- R₇ est un atome d'hydrogène, un groupe alkyle C₁₋₄, ou alkényle C₃₋₄;
- R₈ est un atome d'hydrogène, un groupe alkyle C₁₋₄, carboxyle, carboxamido, ou (CH₂)ₚCO₂R₁₂, dans lequel p est égal à 1 ou à 2, et R₁₂ représente un groupe alkyle C₁₋₆ ou un atome d'hydrogène lorsque R₇ et R₉ sont des atomes d'hydrogène ou des groupes alkyle C₁₋₄;
- R₉ est un atome d'hydrogène, un groupe alkyle C₁₋₄, ou (CH₂)ₚCO₂R¹³, dans lequel p est égal à 1 ou à 2, et R₁₃ est un groupe alkyle C₁₋₆, ou un atome d'hydrogène, à condition que lorsque n est égal à 0, R₅ est un atome d'hydrogène et que de plus, R₇, R₈, et R₉ ne sont pas tous des atomes d'hydrogène;
- R₁₄ et R₁₅ sont indépendamment des atomes d'hydrogène ou des groupes alkyle C₁₋₄, en chaque point, lorsque d est n'est pas égal à 0;
- d est compris entre 0 et 6;
- W est un cycle à 6 atomes, aryle ou hétéroaryle choisi parmi les groupes phényle, pyridyle, ou pyrimidyle non substitué ou substitué par F, E ou D; ou W est un des groupes 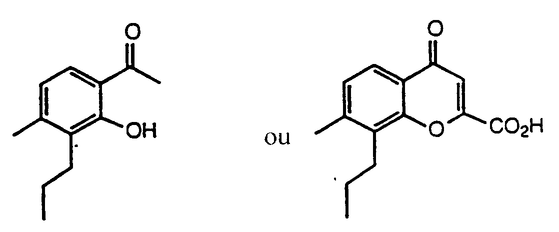
- F est un groupe : 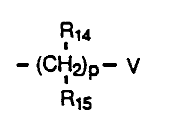 dans lequel R₁₄ et R₁₅ sont indépendamment des atomes d'hydrogène ou des groupes alkyle C₁₋₄,
- p est compris entre 0 et 6;
- V est un atome d'hydrogène, un groupe allkyle C₁₋₄, COR', SO₃H, SO₂H, SO₂NH₂, COCH₂OH, CHOHCH₂OH, ou tétrazolyle, avec R' tel que défini plus haut; et
- E et D sont indépendamment, choisis parmi les groupes H, OH, F, Cl, Br, CF₃, alkyle C₁₋₄, alcoxy C₁₋₄, méthylthio, trifluorométhylthio, NO₂, NH₂, NHalkyle C₁₋₄, ou alkylC₁₋₄CO-;
lequel procédé comprend la réaction d'un composé de formule : 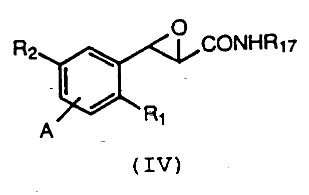 dans lequel :
- R₁ représente un groupe (L)ₐ-(CH₂)_{b}-(T)_{c}-B;
- a est égal à 0 ou à 1;
- b est compris entre 3 et 14;
- c est égal à 0 ou à 1;
- L et T sont, indépendamment, des atomes de soufre , d'oxygène, des groupes CH=CH; C≡C, ou CH₂;
- B est un atome d'hydrogène, un groupe alkyle C₁₋₄, ethinyle, trifluorométhyle, isopropényle, furanyle, thiényle, cyclohexyle, ou phényle, éventuellement monosubstitué par Br, Cl, CF₃, un groupe alcoxy C₁₋₄, alkyle C₁₋₄, méthylthio ou trifluorométhylthio;
- R₂ et A sont choisis indépendamment parmi les groupes H, CF₃, alkyle C₁₋₄, alcoxy C₁₋₄, F, Cl, Br, I OH, NO₂, ou NH₂; ou, lorsque R₁ et A sont des atomes d'hydrogène, R₂ est un groupe (L)ₐ-(CH₂)_{b}-(T)_{c}-B dans lequel a,b c, L, T, et B sont tels que définis ci-dessus; et
- R₁₇ est un atome d'hydrogène, un groupe phényle, benzyle ou alkyle C₁₋₄;
avec l'isopropoxyde de titane et un composé de formule :
R₃-S-M
dans lequel R₃ est tel que défini plus haut, et M représente le lithium, le sodium ou le potassium; et
l'hydrolyse du produit obtenu.

10. Procédé suivant la revendication 9, pour la préparation de l'acide (R*,S*) 2-hydroxy-3-[2-(carboxy)éthylthio]-3-[2-(8-phényl-octyl)phényl]propanoïque.
